# EUROPEAN PATENT SPECIFICATION

(11) **EP 3 856 191 B1**
(45) Date of publication and mention of the grant of the patent: **09.12.2026**
(21) Application number: 19864482.5
(22) Date of filing: 25.09.2019
(51) Int. Cl.: A61K 31/519, A61K 45/06, C07D 487/04, A61P 29/00, A61P 37/06

(54) **PDE1 INHIBITOR FOR USE IN TREATING INFLAMMATORY DISEASES**
PDE1-HEMMER ZUR VERWENDUNG BEI DER BEHANDLUNG VON ENTZÜNDLICHEN ERKRANKUNGEN
INHIBITEUR DE PDE1 POUR SON UTILISATION DANS LE TRAITEMENT DE MALADIES INFLAMMATOIRES

(30) Priority: 25.09.2018 US 201862736324 P
(43) Date of publication of application: 04.08.2021
(73) Proprietor: Intra-Cellular Therapies, Inc., Bedminster, NJ 07921 (US)
(72) Inventor: SNYDER, Gretchen, New York, New York 10016 (US); WENNOGLE, Lawrence P., New York, New York 10016 (US); O'BRIEN, Jennifer, New York, New York 10016 (US); HENDRICK, Joseph, New York, New York 10016 (US)
(74) Representative: Marks & Clerk LLP
(86) International application number: PCT/US2019/053032
(87) International publication number: WO 2020/069043

(56) References cited:
- WO-A1-2018/049417
- WO-A1-2018/049417
- US-A1- 2014 128 353
- US-A1- 2015 017 267
- MARTINEZ FERNANDO O. ET AL: "Genetic programs expressed in resting and IL-4 alternatively activated mouse and human macrophages: similarities and differences", vol. 121, no. 9, 28 February 2013 (2013-02-28), US, pages e57 - e69, XP055919746, ISSN: 0006-4971, Retrieved from the Internet <URL:https://ashpublications.org/blood/article-pdf/121/9/e57/1369390/zh800913000e57.pdf> DOI: 10.1182/blood-2012-06-436212
- SIQUEIRA MIETTO B. ET AL: "Role of IL-10 in Resolution of Inflammation and Functional Recovery after Peripheral Nerve Injury", vol. 35, no. 50, 16 December 2015 (2015-12-16), US, pages 16431 - 16442, XP055919801, ISSN: 0270-6474, Retrieved from the Internet <URL:https://www.jneurosci.org/content/jneuro/35/50/16431.full.pdf> DOI: 10.1523/JNEUROSCI.2119-15.2015
- JACOB R. GREENMYER: "Primary Human Microglia Are Phagocytically Active and Respond to Borrelia burgdorferi With Upregulation of Chemokines and Cytokines", FRONTIERS IN MICROBIOLOGY, vol. 9, 25 April 2018 (2018-04-25), Lausanne, XP093158479, ISSN: 1664-302X, Retrieved from the Internet <URL:https://www.ncbi.nlm.nih.gov/pmc/articles/PMC5996889/pdf/fmicb-09-00811.pdf> DOI: 10.3389/fmicb.2018.00811
- A. BORRAJO: "Important role of microglia in HIV-1 associated neurocognitive disorders and the molecular pathways implicated in its pathogenesis", ANNALS OF MEDICINE, vol. 53, no. 1, 1 January 2021 (2021-01-01), SE, pages 43 - 69, XP093158505, ISSN: 0785-3890, Retrieved from the Internet <URL:https://www.ncbi.nlm.nih.gov/pmc/articles/PMC7877929/pdf/IANN_53_1814962.pdf> DOI: 10.1080/07853890.2020.1814962

## Description

### FIELD OF THE INVENTION

The field relates to inhibitors of phosphodiesterase 1 (PDE1) for use in a method of promoting the resolution of inflammation, for example through the polarization of M1 macrophages to M2 macrophages, for the treatment and prophylaxis of diseases or disorders related to inflammation.

### BACKGROUND OF THE INVENTION

Eleven families of phosphodiesterases (PDEs) have been identified but only PDEs in Family I, the Ca²⁺-calmodulin-dependent phosphodiesterases (CaM-PDEs), are activated by the Ca²⁺-calmodulin and have been shown to mediate the calcium and cyclic nucleotide (e.g. cAMP and cGMP) signaling pathways. These PDEs are therefore active in stimulated conditions when intra-cellular calcium levels rise, leading to increased hydrolysis of cyclic nucleotides. The three known CaM-PDE genes, PDE1A, PDE1B, and PDE1C, are all expressed in central nervous system tissue. In the brain, the predominant expression of PDE1A is in the cortex and neostriatum, PDE1B is expressed in the neostriatum, prefrontal cortex, hippocampus, and olfactory tubercle, and PDE1C is more ubiquitously expressed.

PDE4 is the major cAMP-metabolizing enzyme found in inflammatory and immune cells, and PDE4 inhibitors are of interest as anti-inflammatory drugs. PDE1, however, has not been thought to play a major role in the inflammatory response, although PDE-1 is induced in monocyte-to-macrophage differentiation mediated by the cytokine granulocyte-macrophage colony-stimulating factor (GM-CSF). The PDE1 inhibitor vinpocetine has been shown to be anti-inflammatory, but the anti-inflammatory action of vinpocetine is believed to be caused by a direct inhibition of the IκB kinase complex (IKK) rather than PDE blockade.

Macrophages have a central role in maintaining homeostasis and mediating inflammation in the body. Macrophages are capable of polarization by which a macrophage expresses different functional programs in response to micro environmental signals. There are several activated forms of macrophages, but the two main groups are designated as M1 and M2. M1 macrophages, also referred to as "classically activated macrophages," are activated by LPS and IFN-gamma, and secrete high levels of IL-12 and low levels of IL-10 for a pro-inflammatory effect. In contrast, the M2 designation, also referred to as "alternatively activated macrophages," broadly refers to macrophages that function in constructive processes like wound healing and tissue repair, and those that turn off damaging immune system activation by producing anti-inflammatory cytokines like IL-10. M2 macrophages produce high levels of IL-10, TGF-beta and low levels of IL-12. Prolonged M1 type of macrophages is harmful for the organism and that is why tissue repair and restoration is necessary.

When tissues are challenged by pathogens, inflammatory monocytes in circulation are recruited and differentiated into macrophages. Generally, macrophages are polarized toward an M1 phenotype in the early stages of bacterial infection. When the bacteria are recognized by pathogen recognition receptors, macrophages are activated and produce a large amount of pro-inflammatory mediators including TNF-α, IL-1, and nitric oxide (NO), which kill the invading organisms and activate the adaptive immunity. For example, this mechanism has been considered to be involved in infection with *Salmonella typhi, Salmonella typhimurium, Listeria monocytogenes,* and the early phases of infection with *Mycobacterium tuberculosis, Mycobacterium ulcerans,* and *Mycobacterium avium.* If macrophage-mediated inflammatory response cannot be quickly controlled, a cytokine storm is formed, thereby contributing to the pathogenesis of severe sepsis. In order to counteract the excessive inflammatory response, macrophages undergo apoptosis or polarize to an M2 phenotype to protect the host from excessive injury and facilitate wound healing.

Macrophage polarization is also involved in virus infection, in which M2 phenotype macrophages can also suppress inflammation and promote tissue healing. Influenza virus augments the phagocytic function of human macrophages, which is a major feature of M2 phenotype, to clear apoptotic cells and accelerate the resolution of inflammation. In severe acute respiratory syndrome (SARS)-Cov infection, M2 phenotype macrophages are critical to regulate immune response and protect host from the long-term progression to fibrotic lung disease by a STAT dependent pathway. In addition, severe respiratory syncytial virus (RSV) induced bronchiolitis is closely associated with mixed M1 and M2 macrophages.

Many viruses elicit mechanisms to adapt and modulate macrophage polarization. In human monocyte-derived macrophages, HIV-1 infection has been observed to skew cells toward a M1-like status, which correlates with downregulation of M2-status markers (i.e., CD163, CD206, CCL18, and IL-10) and increased secretion of M1-associated chemokines including CCL3, CCL4, and CCL5.

Macrophage polarization has also been shown to play a significant role in various inflammatory diseases and disorders, such as nonalcoholic steatohepatitis (NASH), atherosclerosis, metabolic disease, systemic lupus erythematosus, among many others.

It has not been previously shown that PDE1 has a significant role in mediating resolution of inflammation, or that it would have a significant effect on inflammatory diseases. Inflammatory processes in general, and diseases and disorders related to inflammation, are numerous, and the mechanisms and actions are still not well understood. Currently, there is a largely unmet need for an effective way of treating inflammation and inflammatory related diseases and disorders, especially with regard to inflammation occurring in the brain.

### BRIEF DESCRIPTION OF DRAWINGS

Figure 1 depicts the number of leukocytes detected at the site of inflammation following sterile insult when treated with Compound 1.
Figure 2A depicts the number of macrophages detected at the site of inflammation following sterile insult when treated with Compound 1.
Figure 2B depicts the number of macrophages expressed as percent of total leukocytes detected at the site of inflammation following sterile insult when treated with Compound 1.
Figure 3A depicts the number of neutrophils detected at the site of inflammation following sterile insult when treated with Compound 1.
Figure 3B depicts the amount of neutrophils expressed as percent of total leukocytes detected at the site of inflammation following sterile insult when treated with Compound 1.
Figure 4A depicts the amount of M1 macrophages expressed as a percentage of total macrophages detected at the site of inflammation following sterile insult when treated with Compound 1.
Figure 4B depicts the amount of M2 macrophages expressed as a percentage of total macrophages detected at the site of inflammation following sterile insult when treated with Compound 1.
Figure 5A depicts the number of M1 macrophages detected at the site of inflammation in the M2 activation state following sterile insult when treated with Compound 1.
Figure 5B depicts the number of M2 macrophages detected at the site of inflammation following sterile insult when treated with Compound 1.
Figure 6A depicts the mean fluorescent intensity (MFI) of CD38 expression on macrophage populations detected at the site of inflammation following sterile insult when treated with Compound 1.
Figure 6B depicts the mean fluorescent intensity (MFI) of CD38 expression on macrophage populations detected at the site of inflammation following sterile insult when treated with Compound 1.
Figure 7 depicts cytokine production in plasma in test subjects following sterile insult when treated with Compound 1.
Figure 8 depicts the number of macrophages in the M1 activation state detected at the site of inflammation following sterile insult when treated with Compound 2.
Figure 9 depicts the number of macrophages in the M2 activation state detected at the site of inflammation following sterile insult when treated with Compound 2.
Figure 10 (not according to the claimed invention and present for illustration purposes only) depicts the results of Compound 1 on the motility of BV2 cells in a microglia chemotaxis assay.
Figure 11A depicts the amount of CD80+ macrophages expressed as a percentage of total macrophages detected at the site of inflammation.
Figure 11B depicts the amount of iNOS+ macrophages expressed as a percentage of total macrophages detected at the site of inflammation.
Figure 12A depicts the amount of Arg1+ macrophages expressed as a percentage of total macrophages detected at the site of inflammation.

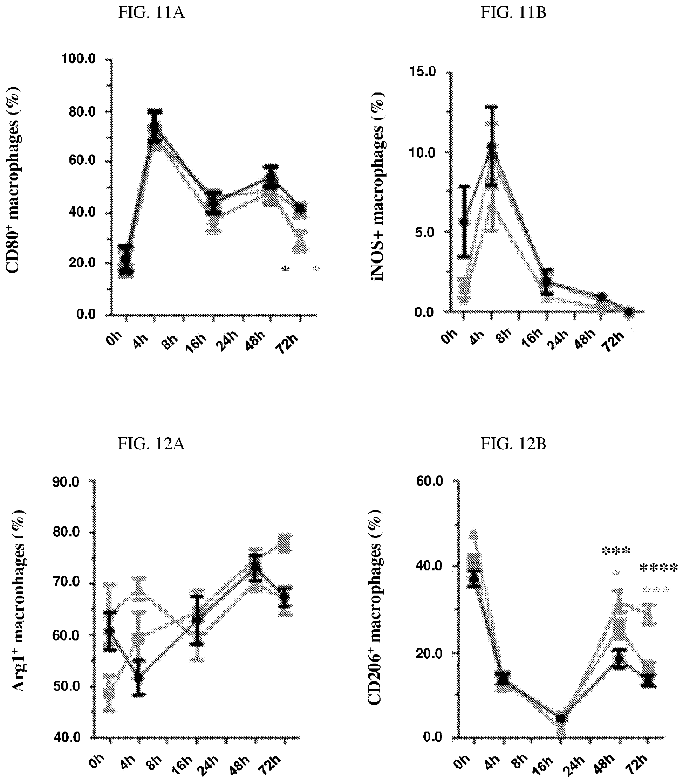

Figure 12A depicts the amount of CD206+ macrophages expressed as a percentage of total macrophages detected at the site of inflammation.

### SUMMARY OF THE INVENTION

The invention is set out in the appended set of claims.

References herein to methods of treatment by therapy are to be interpreted as references to compounds and pharmaceutical compositions described herein for use in such methods.

Surprisingly, we have discovered that PDE1 mediates the expression of certain pro-inflammatory cytokines and chemokines and that PDE1 inhibitors have specific anti-inflammatory effects. Inhibition of PDE1 regulates inflammatory activity in macrophages, reducing expression of pro-inflammatory genes, thereby providing novel treatments for a variety of disorders and conditions characterized by macrophage mediation.

Positive regulation of inflammatory resolution responses in macrophages by elevated intracellular cyclic nucleotide levels provides a promising area for therapeutic intervention. It is known that PDE1B is present in monocytes and involved in the differentiation into macrophage via growth factor activation signals such as GM-CSF. Bender AT, et al., Selective up-regulation of PDE1B2 upon monocyte-to-macrophage differentiation, Proc Natl Acad Sci U S A. 2005 Jan 11; 102(2): 497-502. Cyclic guanosine monophosphate (cGMP) has been demonstrated to be a key modulator of the differentiation pathways in macrophages. cGMP also plays a role in modulation of inflammatory processes, such as inducible NO synthase induction and TNF-α release. Therefore, the marked up-regulation of PDE1B may be critical in the regulation of these processes in differentiated macrophages. This suggests that PDE1 inhibitors, such as those disclosed herein, may prove beneficial in diseases associated with, for example, inflammation disorders relating to macrophage activation.

In one embodiment, therefore, the invention provides using various PDE1 inhibitory compounds to treat inflammation, and/or diseases or disorders related to inflammation according to the appended claims. Without being bound by theory, one possible mechanism for this activity is that inhibition of PDE1B may affect macrophage activation in the blood and/or microglial activation in the CNS, so as to reduce M1 activation and the release of pro-inflammatory cytokines, and to promote the polarization of macrophages to M2 type through the up-regulation of anti-inflammatory cytokines such as IL-10. Discussion of the treatment of and prophylaxis of inflammation and/or diseases or disorders related to inflammation as they relate to the microglia e.g., neuroinflammation, is discussed in International Publication WO 2018/049417.

The regulation of M1 to M2 type activation in macrophages is central to inflammatory pathways in a number of disorders. The role of M1 to M2 polarization in macrophages is important in a number of inflammatory-related disorders including bacterial infections (e.g., *Salmonella typhi, Salmonella typhimurium, Listeria monocytogenes, Mycobacterium tuberculosis, Mycobacterium ulcerans,* and *Mycobacterium avium* infections); viral infections (e.g., African Swine Fever Virus, Classical Swine Fever Virus, Dengue Virus, Foot and Mouth Disease Virus, Human Immunodeficiency Virus (HIV) (e.g., HIV1), Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, Viral Hepatitis (e.g., Hepatitis A, Hepatitis B, Hepatitis C)); parasitic infestations (e.g., *Taenia crassiceps, Toxoplasma gondii, Leishmania infantum, Schistosoma mansoni* infestations); atopic dermatitis; pneumonia; cardiovascular diseases, such as atherosclerosis; obesity and insulin resistance; asthma; pulmonary fibrosis; chronic obstructive pulmonary disease (COPD); neuropathic pain; stroke; diabetes; sepsis; nonalcoholic steatoheptatitis (NASH); autoimmune hepatitis; systemic lupus erythematosus (SLE); wound healing; pleurisy; peritonitis; and cystic fibrosis.

Targeted inhibition of PDE1 with a compound of the present invention is believed to affect macrophage activation and promote production of anti-inflammatory cytokines and factors involved in resolution of macrophage mediated inflammation.

The invention provides a PDE1 inhibitor or a pharmaceutical composition comprising a PDE1 inhibitor for use in a method of promoting resolution of inflammation for the treatment or prophylaxis of inflammation or disease associated with inflammation, the method comprising administering the PDE1 inhibitor or pharmaceutical composition to a patient in need thereof, wherein the patient is suffering from a disease or disorder mediated by macrophages, selected from bacterial infections selected from Salmonella typhi, Salmonella typhimurium, Listeria monocytogenes, Mycobacterium tuberculosis, Mycobacterium ulcerans, and Mycobacterium avium infections; viral infections selected from African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, Viral Hepatitis (e.g., Hepatitis A, Hepatitis B, Hepatitis C); parasitic infestations (e.g., Taenia crassiceps, Toxoplasma gondii, Leishmania infantum, Schistosoma mansoni infestations); atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis, whereby the PDE1 inhibitor is of Formulas I, Ia, II, III and/or IV as herein described.

Further embodiments of the invention are set forth or evident from the detailed description below and the examples herein.

### DETAILED DESCRIPTION OF THE INVENTION

### Compounds for use in the methods of the invention

The PDE1 inhibitors for use in the methods of treatment and prophylaxis described herein are optionally substituted 7,8-dihydro-imidazo[1,2-a]pyrazolo[4,3-e]pyrimidin-4-one compounds and 7,8,9-trihydro-[1H or 2H]-pyrimido [1,2-a]pyrazolo[4,3-e]pyrimidin-4(5H)-one compounds, in free or pharmaceutically acceptable salt form.

In yet another embodiment the invention provides that the PDE1 inhibitors for use in the methods of treatment and prophylaxis described herein are compounds of Formula I: 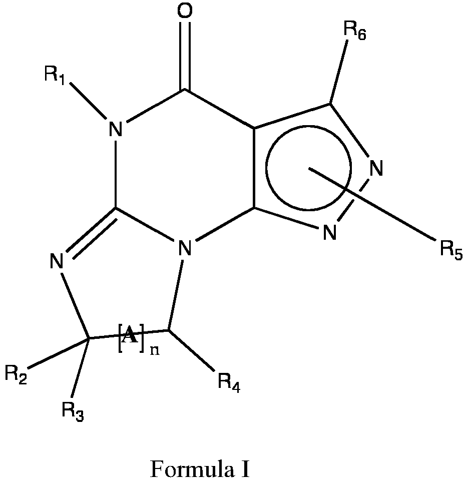 wherein
(i) R₁ is H or C₁₋₄ alkyl (e.g., methyl);
(ii) R₄ is H or C₁₋₄ alkyl and R₂ and R₃ are, independently, H or C₁₋₄ alkyl (e.g., R₂ and R₃ are both methyl, or R₂ is H and R₃ is isopropyl), aryl, heteroaryl, (optionally hetero)arylalkoxy, or (optionally hetero)arylalkyl; or
   R₂ is H and R₃ and R₄ together form a di-, tri- or tetramethylene bridge (pref. wherein the R₃ and R₄ together have the *cis* configuration, e.g., where the carbons carrying R₃ and R₄ have the R and S configurations, respectively);
(iii) R₅ is a substituted heteroarylalkyl, e.g., substituted with haloalkyl;
   or R₅ is attached to one of the nitrogens on the pyrazolo portion of Formula I and is a moiety of Formula A 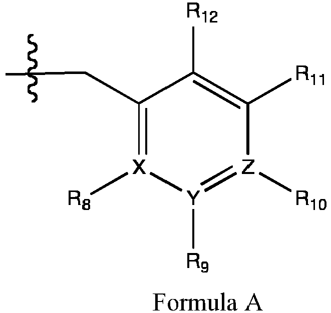
   wherein X, Y and Z are, independently, N or C, and R₈, R₉, R₁₁ and R₁₂ are independently H or halogen (e.g., Cl or F), and R₁₀ is halogen, alkyl, cycloalkyl, haloalkyl (e.g., trifluoromethyl), aryl (e.g., phenyl), heteroaryl (e.g., pyridyl (for example pyrid-2-yl) optionally substituted with halogen, or thiadiazolyl (e.g., 1,2,3-thiadiazol-4-yl)), diazolyl, triazolyl, tetrazolyl, arylcarbonyl (e.g., benzoyl), alkylsulfonyl (e.g., methylsulfonyl), heteroarylcarbonyl, or alkoxycarbonyl; provided that when X, Y, or Z is nitrogen, R₈, R₉, or R₁₀, respectively, is not present; and
(iv) R₆ is H, alkyl, aryl, heteroaryl, arylalkyl (e.g., benzyl), arylamino (e.g., phenylamino), heteroarylamino, N,N-dialkylamino, N,N-diarylamino, or N-aryl-N-(arylakyl)amino (e.g., N-phenyl-N-(1,1'-biphen-4-ylmethyl)amino); and
(v) n=0 or 1;
(vi) when n=1, A is -C(R₁₃R₁₄)-
   wherein R₁₃ and R₁₄, are, independently, H or C₁₋₄ alkyl, aryl, heteroaryl, (optionally hetero)arylalkoxy or (optionally hetero)arylalkyl;
   in free or salt form, including its enantiomers, diastereoisomers and racemates.

In another embodiment the invention provides that the PDE1 inhibitors for use in the methods as described herein are Formula 1a: 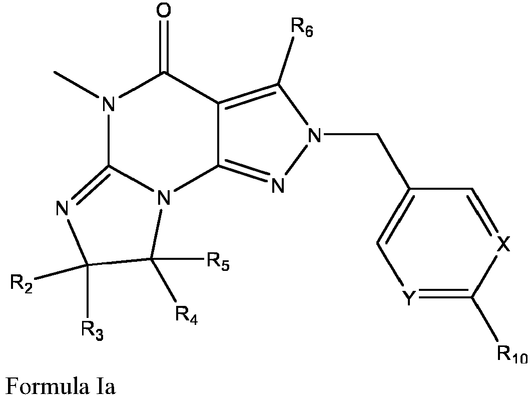 wherein
(i) R₂ and R₅ are independently H or hydroxy and R₃ and R₄ together form a tri- or tetra-methylene bridge [pref. with the carbons carrying R₃ and R₄ having the R and S configuration respectively]; or R₂ and R₃ are each methyl and R₄ and R₅ are each H; or R₂, R₄ and R₅ are H and R₃ is isopropyl [pref. the carbon carrying R₃ having the R configuration];
(ii) R₆ is (optionally halo- or hydroxy-substituted) phenylamino, (optionally halo- or hydroxy-substituted) benzylamino, C₁₋₄alkyl, or C₁₋₄alkyl sulfide; for example, phenylamino or 4-fluorophenylamino;
(iii) R₁₀ is C₁₋₄alkyl, methylcarbonyl, hydroxyethyl, carboxylic acid, sulfonamide, (optionally halo- or hydroxy-substituted) phenyl, (optionally halo- or hydroxy-substituted) pyridyl (for example 6-fluoropyrid-2-yl), or thiadiazolyl (e.g., 1,2,3-thiadiazol-4-yl); and X and Y are independently C or N,
in free or pharmaceutically acceptable salt form, including its enantiomers, diastereoisomers and racemates.

In another embodiment the invention provides that the PDE1 inhibitors for use in the methods of treatment and prophylaxis described herein are compounds of Formula II: 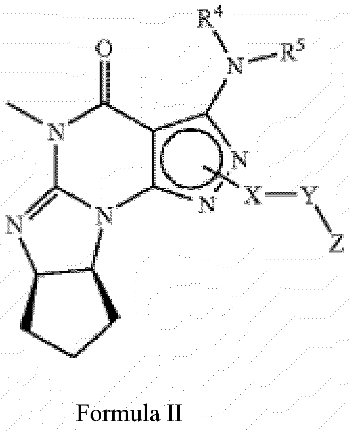
(i) X is C₁₋₆alkylene (e.g., methylene, ethylene or prop-2-yn-1-ylene);
(ii) Y is a single bond, alkynylene (e.g., -C≡C-), arylene (e.g., phenylene) or heteroarylene (e.g., pyridylene);
(iii) Z is H, aryl (e.g., phenyl), heteroaryl (e.g., pyridyl, e.g., pyrid-2-yl), halo (e.g., F, Br, Cl), haloC₁₋₆alkyl (e.g., trifluoromethyl), -C(O)-R¹, -N(R²)(R³), or C₃₋₇cycloalkyl optionally containing at least one atom selected from a group consisting of N or O (e.g., cyclopentyl, cyclohexyl, tetrahydro-2H-pyran-4-yl, or morpholinyl);
(iv) R' is C₁₋₆alkyl, haloC₁₋₆alkyl, -OH or -OC₁₋₆alkyl (e.g., -OCH₃);
(v) R² and R³ are independently H or C₁₋₆alkyl;
(vi) R⁴ and R⁵ are independently H, C₁₋₆alky or aryl (e.g., phenyl) optionally substituted with one or more halo (e.g., fluorophenyl, e.g., 4-fluorophenyl), hydroxy (e.g., hydroxyphenyl, e.g., 4-hydroxyphenyl or 2-hydroxyphenyl) or C₁₋₆alkoxy;
(vii) wherein X, Y and Z are independently and optionally substituted with one or more halo (e.g., F, Cl or Br), C₁₋₆alkyl (e.g., methyl), haloC₁₋₆alkyl (e.g., trifluoromethyl), for example, Z is heteroaryl, e.g., pyridyl substituted with one or more halo (e.g., 6-fluoropyrid-2-yl, 5-fluoropyrid-2-yl, 6-fluoropyrid-2-yl, 3-fluoropyrid-2-yl, 4-fluoropyrid-2-yl, 4,6-dichloropyrid-2-yl), haloC₁₋₆alkyl (e.g., 5-trifluoromethylpyrid-2-yl) or C₁₋₆-alkyl (e.g., 5-methylpyrid-2-yl), or Z is aryl, e.g., phenyl, substituted with one or more halo (e.g., 4-fluorophenyl),
in free or salt form.

In yet another embodiment the invention provides that the PDE1 inhibitors for use in the methods of treatment and prophylaxis described herein are Formula III: 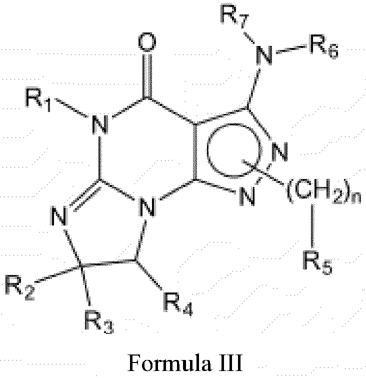 wherein
(i) R1 is H or C₁₋₄ alkyl (e.g., methyl or ethyl);
(ii) R₂ and R₃ are independently H or C₁₋₆ alkyl (e.g., methyl or ethyl);
(iii) R₄ is H or C₁₋₄ alkyl (e.g., methyl or ethyl);
(iv) R₅ is aryl (e.g., phenyl) optionally substituted with one or more groups independently selected from -C(=O)-C₁₋₆ alkyl (e.g., -C(=O)-CH₃) and C₁₋₆ -hydroxyalkyl (e.g., 1-hydroxyethyl);
(v) R₆ and R₇ are independently H or aryl (e.g., phenyl) optionally substituted with one or more groups independently selected from C₁₋₆ alkyl (e.g., methyl or ethyl) and halogen (e.g., F or Cl), for example unsubstituted phenyl or phenyl substituted with one or more halogen (e.g., F) or phenyl substituted with one or more C₁₋₆ alkyl and one or more halogen or phenyl substituted with one C₁₋₆ alkyl and one halogen, for example 4-fluorophenyl or 3,4-difluorophenyl or 4-fluoro-3-methylphenyl; and
(vi) n is 1, 2, 3, or 4,
in free or salt form.

In yet another embodiment the invention provides that the PDE1 inhibitors for use in the methods of treatment and prophylaxis described herein are Formula IV 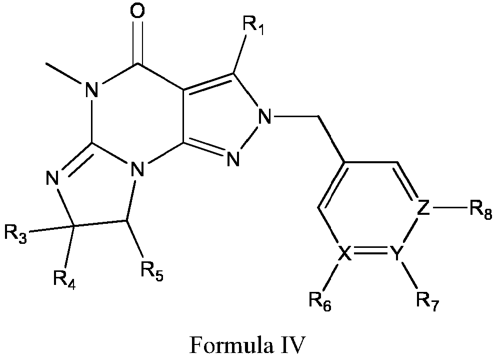 in free or salt form, wherein
(i) R₁ is C₁₋₄alkyl (e.g., methyl or ethyl), or -NH(R₂), wherein R₂ is phenyl optionally substituted with halo (e.g., fluoro), for example, 4-fluorophenyl;
(ii) X, Y and Z are, independently, N or C;
(iii) R₃, R₄ and R₅ are independently H or C₁₋₄alkyl (e.g., methyl); or R₃ is H and R₄ and R₅ together form a tri-methylene bridge (pref. wherein the R₄ and R₅ together have the cis configuration, e.g., where the carbons carrying R₄ and R₅ have the R and S configurations, respectively),
(iv) R₆, R₇ and R₈ are independently:
   H,
   C₁₋₄alkyl (e.g., methyl),
   pyrid-2-yl substituted with hydroxy, or
   -S(O)₂-NH₂;
(v) Provided that when X, Y and/or Z are N, then R₆, R₇ and/or R₈, respectively, are not present; and when X, Y and Z are all C, then at least one of R₆, R₇ or R₈ is -S(O)₂-NH₂ or pyrid-2-yl substituted with hydroxy.

In one embodiment, the present disclosure provides for administration of a PDE1 inhibitor for use in the methods described herein (e.g., a compound according to Formulas I, Ia, II, III, and/or IV), wherein the inhibitor is a compound according to the following: 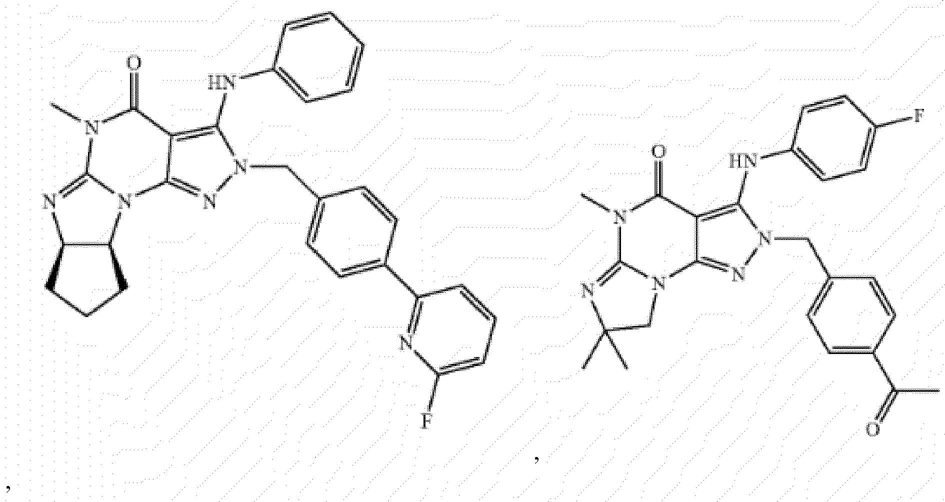 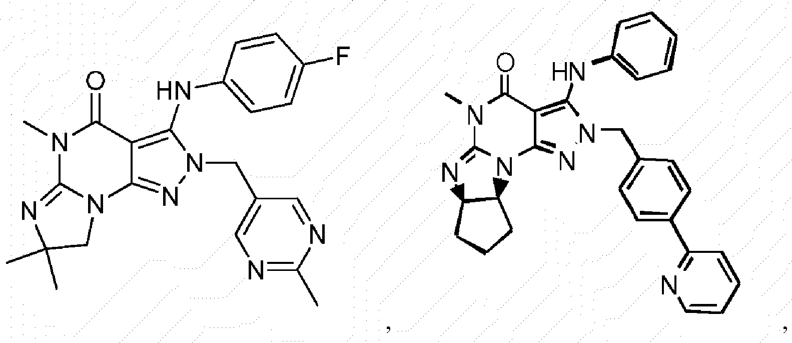 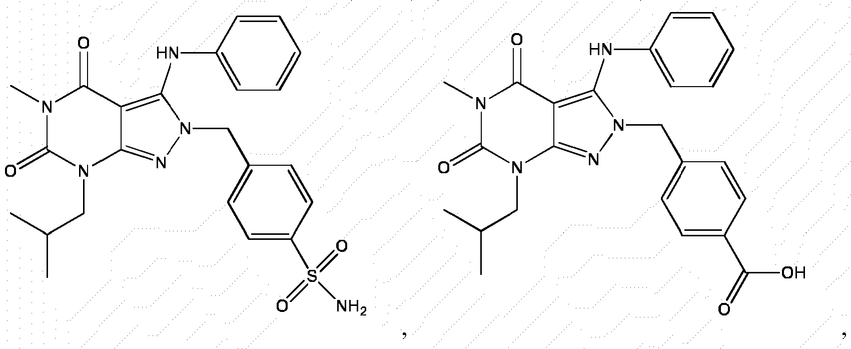 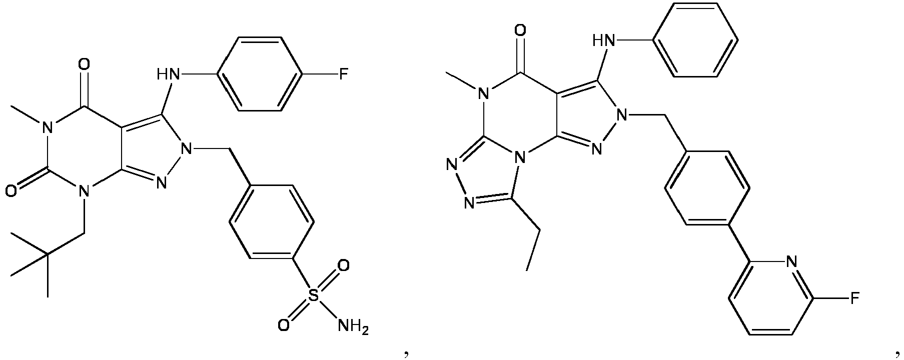 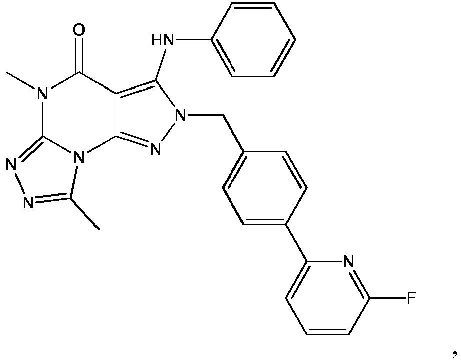 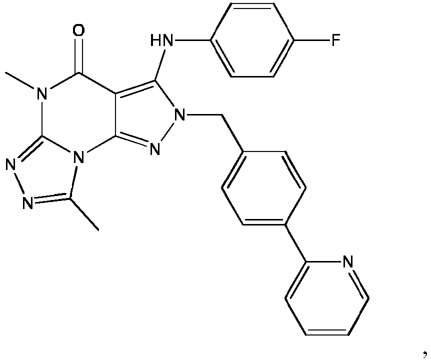 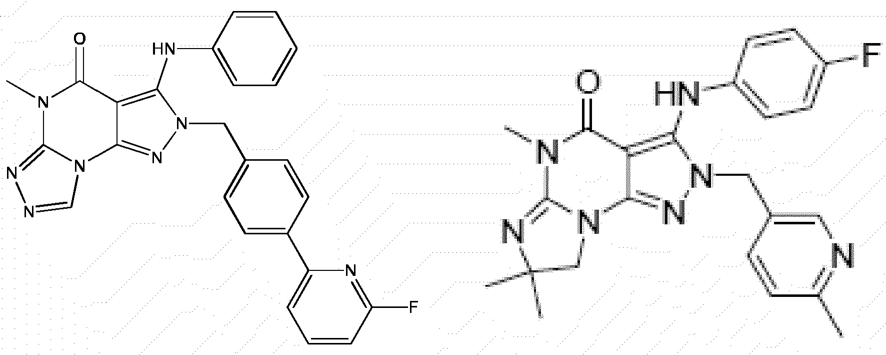 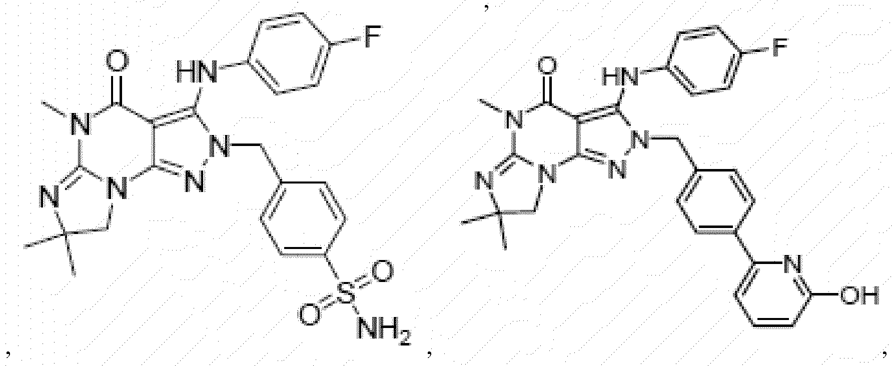 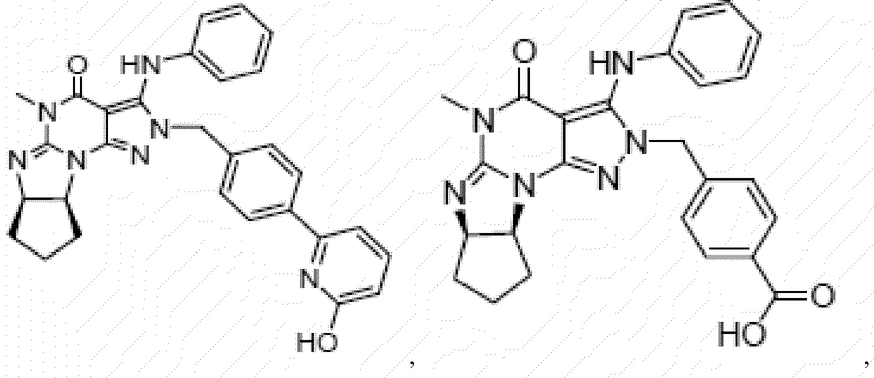 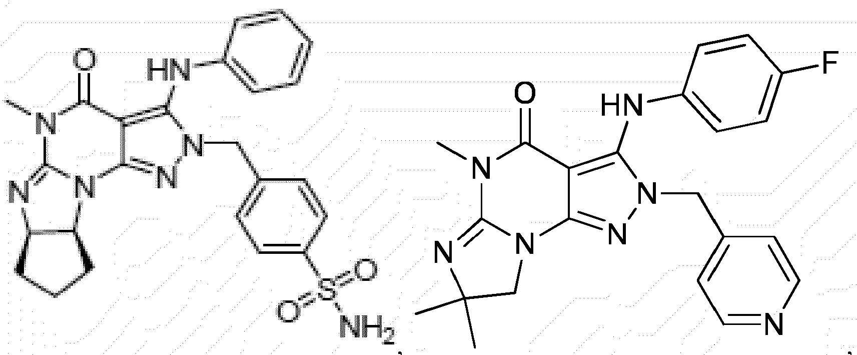 or

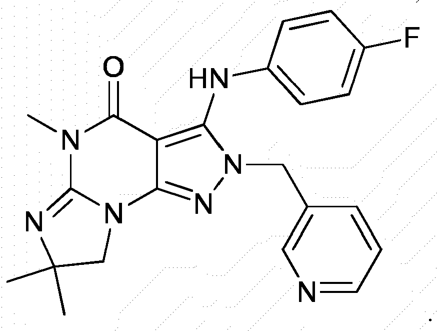

In one embodiment the invention provides administration of a PDE1 inhibitor for treatment or prophylaxis of inflammation or an inflammatory related disease or disorder, as defined in claim 1, wherein the inhibitor is a compound according to the following: 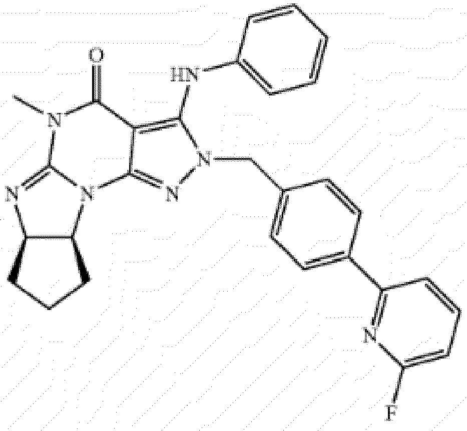 in free or pharmaceutically acceptable salt form.

In still another embodiment, the invention provides administration of a PDE1 inhibitor for treatment or prophylaxis of inflammation or an inflammatory related disease or disorder, as defined in claim 1, wherein the inhibitor is a compound according to the following: 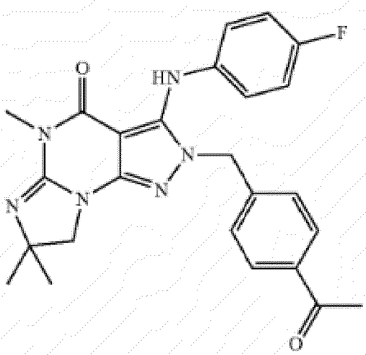 in free or pharmaceutically acceptable salt form.

In still another embodiment, the invention provides administration of a PDE1 inhibitor for treatment or prophylaxis of inflammation or an inflammatory related disease or disorder, as defined in claim 1, wherein the inhibitor is a compound according to the following: 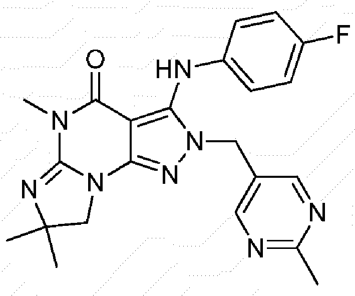 in free or pharmaceutically acceptable salt form.

In still another embodiment, the invention provides administration of a PDE1 inhibitor for treatment or prophylaxis of inflammation or an inflammatory related disease or disorder, as defined in claim 1, wherein the inhibitor is a compound according to the following: 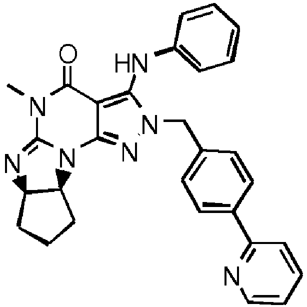 in free or pharmaceutically acceptable salt form.

In one embodiment, selective PDE1 inhibitors of the any of the preceding formulae (e.g., Formulas I, Ia, II, III, and/or IV) are compounds that inhibit phosphodiesterase-mediated (e.g., PDE1-mediated, especially PDE1B-mediated) hydrolysis of cGMP, e.g., the preferred compounds have an IC₅₀ of less than 1µM, preferably less than 500 nM, preferably less than 50 nM, and preferably less than 5nM in an immobilized-metal affinity particle reagent PDE assay, in free or salt form.

If not otherwise specified or clear from context, the following terms herein have the following meanings:
"Alkyl" as used herein is a saturated or unsaturated hydrocarbon moiety, preferably saturated, preferably having one to six carbon atoms, which may be linear or branched, and may be optionally mono-, di- or tri- substituted, e.g., with halogen (e.g., chloro or fluoro), hydroxy, or carboxy.
"Cycloalkyl" as used herein is a saturated or unsaturated nonaromatic hydrocarbon moiety, preferably saturated, preferably comprising three to nine carbon atoms, at least some of which form a nonaromatic mono- or bicyclic, or bridged cyclic structure, and which may be optionally substituted, e.g., with halogen (e.g., chloro or fluoro), hydroxy, or carboxy. Wherein the cycloalkyl optionally contains one or more atoms selected from N and O and/or S, said cycloalkyl may also be a heterocycloalkyl.
"Heterocycloalkyl" is, unless otherwise indicated, saturated or unsaturated nonaromatic hydrocarbon moiety, preferably saturated, preferably comprising three to nine carbon atoms, at least some of which form a nonaromatic mono- or bicyclic, or bridged cyclic structure, wherein at least one carbon atom is replaced with N, O or S, which heterocycloalkyl may be optionally substituted, e.g., with halogen (e.g., chloro or fluoro), hydroxy, or carboxy.
"Aryl" as used herein is a mono or bicyclic aromatic hydrocarbon, preferably phenyl, optionally substituted, e.g., with alkyl (e.g., methyl), halogen (e.g., chloro or fluoro), haloalkyl (e.g., trifluoromethyl), hydroxy, carboxy, or an additional aryl or heteroaryl (e.g., biphenyl or pyridylphenyl).
"Heteroaryl" as used herein is an aromatic moiety wherein one or more of the atoms making up the aromatic ring is sulfur or nitrogen rather than carbon, e.g., pyridyl or thiadiazolyl, which may be optionally substituted, e.g., with alkyl, halogen, haloalkyl, hydroxy or carboxy.

Compounds of the Invention are optionally substituted 7,8-dihydro-imidazo[1,2-a]pyrazolo[4,3-e]pyrimidin-4-one compounds and 7,8,9-trihydro-[1H or 2H]-pyrimido [1,2-a]pyrazolo[4,3-e]pyrimidin-4(5H)-one compounds, in free or pharmaceutically acceptable salt form, which are Compounds of Formulas I, Ia, II, III, and/or IV, and which may exist in free or salt form, e.g., as acid addition salts. In this specification unless otherwise indicated, language such as "Compounds of the Invention" is to be understood as embracing the compounds in any form, for example free or acid addition salt form, or where the compounds contain acidic substituents, in base addition salt form. The Compounds of the Invention are intended for use as pharmaceuticals, therefore pharmaceutically acceptable salts are preferred. Salts which are unsuitable for pharmaceutical uses may be useful, for example, for the isolation or purification of free Compounds of the Invention or their pharmaceutically acceptable salts, are therefore also included.

Compounds of the disclosure may in some cases also exist in prodrug form (not claimed). A prodrug form is compound which converts in the body to a Compound of the Invention. For example when the Compounds of the Invention contain hydroxy or carboxy substituents, these substituents may form physiologically hydrolysable and acceptable esters. As used herein, "physiologically hydrolysable and acceptable ester" means esters of Compounds of the Invention which are hydrolysable under physiological conditions to yield acids (in the case of Compounds of the Invention which have hydroxy substituents) or alcohols (in the case of Compounds of the Invention which have carboxy substituents) which are themselves physiologically tolerable at doses to be administered. Therefore, wherein the Compound of the Invention contains a hydroxy group, for example, Compound-OH, the acyl ester prodrug of such compound, i.e., Compound-O-C(O)-C₁₋₄alkyl, can hydrolyze in the body to form physiologically hydrolysable alcohol (Compound-OH) on the one hand and acid on the other (e.g., HOC(O)-C₁₋₄alkyl). Alternatively, wherein the Compound of the Invention contains a carboxylic acid, for example, Compound-C(O)OH, the acid ester prodrug of such compound, Compound-C(O)O-C₁₋₄alkyl can hydrolyze to form Compound-C(O)OH and HO-C₁₋₄alkyl. As will be appreciated the term thus embraces conventional pharmaceutical prodrug forms.

In another embodiment, the invention further provides a pharmaceutical composition comprising a Compound of the Invention, in free or pharmaceutically acceptable salt form, in admixture with a pharmaceutically acceptable carrier, for use as an anti-inflammatory agent, in accordance with claim 1.

Compounds of the disclosure may in some cases also exist in prodrug form (not claimed). A prodrug form is compound which converts in the body to a Compound of the Invention. For example when the Compounds of the Invention contain hydroxy or carboxy substituents, these substituents may form physiologically hydrolysable and acceptable esters. As used herein, "physiologically hydrolysable and acceptable ester" means esters of Compounds of the Invention which are hydrolysable under physiological conditions to yield acids (in the case of Compounds of the Invention which have hydroxy substituents) or alcohols (in the case of Compounds of the Invention which have carboxy substituents) which are themselves physiologically tolerable at doses to be administered. Therefore, wherein the Compound of the Invention contains a hydroxy group, for example, Compound-OH, the acyl ester prodrug of such compound, i.e., Compound-O-C(O)-C₁₋₄alkyl, can hydrolyze in the body to form physiologically hydrolysable alcohol (Compound-OH) on the one hand and acid on the other (e.g., HOC(O)-C₁₋₄alkyl). Alternatively, wherein the Compound of the Invention contains a carboxylic acid, for example, Compound-C(O)OH, the acid ester prodrug of such compound, Compound-C(O)O-C₁₋₄alkyl can hydrolyze to form Compound-C(O)OH and HO-C₁₋₄alkyl. As will be appreciated the term thus embraces conventional pharmaceutical prodrug forms.

In another embodiment, the invention further provides a pharmaceutical composition comprising a Compound of the Invention, in free or pharmaceutically acceptable salt **form,** in admixture with a pharmaceutically acceptable carrier, for use as an anti-inflammatory agent in accordance with claim 1.

### Methods of Making Compounds of the Invention

The compounds of the Invention and their pharmaceutically acceptable salts may be made using the methods as described and exemplified herein and by methods similar thereto and by methods known in the chemical art. Such methods include, but not limited to, those described below. If not commercially available, starting materials for these processes may be made by procedures, which are selected from the chemical art using techniques which are similar or analogous to the synthesis of known compounds.

Various starting materials and/or Compounds of the Invention may be prepared using methods described in US 2008-0188492 A1, US 2010-0173878 A1, US 2010-0273754 A1, US 2010-0273753 A1, WO 2010/065153, WO 2010/065151, WO 2010/065151, WO 2010/065149, WO 2010/065147, WO 2010/065152, WO 2011/153129, WO 2011/133224, WO 2011/153135, WO 2011/153136, WO 2011/153138, and US Pat. 9,073,936.

The Compounds of the Invention include their enantiomers, diastereoisomers and racemates, as well as their polymorphs, hydrates, solvates and complexes. Some individual compounds within the scope of this invention may contain double bonds. Representations of double bonds in this invention are meant to include both the E and the Z isomer of the double bond. In addition, some compounds within the scope of this invention may contain one or more asymmetric centers. This invention includes the use of any of the optically pure stereoisomers as well as any combination of stereoisomers.

It is also intended that the Compounds of the Invention encompass their stable and unstable isotopes. Stable isotopes are nonradioactive isotopes which contain one additional neutron compared to the abundant nuclides of the same species (i.e., element). It is expected that the activity of compounds comprising such isotopes would be retained, and such compound would also have utility for measuring pharmacokinetics of the non-isotopic analogs. For example, the hydrogen atom at a certain position on the Compounds of the Invention may be replaced with deuterium (a stable isotope which is non-radioactive). Examples of known stable isotopes include, but not limited to, deuterium, ¹³ C, ¹⁵ N, ¹⁸ O. Alternatively, unstable isotopes, which are radioactive isotopes which contain additional neutrons compared to the abundant nuclides of the same species (i.e., element), e.g., ¹²³I, ¹³¹I, ¹²⁵I, ¹¹C, ¹⁸F, may replace the corresponding abundant species of I, C and F. Another example of useful isotope of the compound of the invention is the ¹¹C isotope. These radio isotopes are useful for radio-imaging and/or pharmacokinetic studies of the compounds of the invention.

Melting points are uncorrected and (dec) indicates decomposition. Temperature are given in degrees Celsius (°C); unless otherwise stated, operations are carried out at room or ambient temperature, that is, at a temperature in the range of 18-25 °C. Chromatography means flash chromatography on silica gel; thin layer chromatography (TLC) is carried out on silica gel plates. NMR data is in the delta values of major diagnostic protons, given in parts per million (ppm) relative to tetramethylsilane (TMS) as an internal standard. Conventional abbreviations for signal shape are used. Coupling constants (J) are given in Hz. For mass spectra (MS), the lowest mass major ion is reported for molecules where isotope splitting results in multiple mass spectral peaks. Solvent mixture compositions are given as volume percentages or volume ratios. In cases where the NMR spectra are complex, only diagnostic signals are reported.

### Methods of using Compounds of the Invention

The Compounds of the Invention are useful in the treatment of inflammatory diseases or conditions, particularly inflammatory diseases or conditions. Therefore, administration or use of a preferred PDE1 inhibitor as described herein, e.g., a PDE1 inhibitor as hereinbefore described, e.g., a Compound of Formulas I, Ia, II, III, and/or IV provides a means to regulate inflammation (e.g., prevent, reduce, and/or reverse inflammation, and diseases or disorders related to inflammation), and in certain embodiments provide a treatment for various inflammatory diseases and disorders.

For example, in exemplary embodiments of the invention, a method (Method 1) of claim 1 of promoting resolution of inflammation for the treatment or prophylaxis of inflammation or disease associated with inflammation comprising administering an effective amount of a specific inhibitor of phosphodiesterase type I (PDE1), to a patient in need thereof, in which:
1.1. Method 1 wherein the patient is suffering from inflammation and/or a disease or disorder mediated by macrophage activation.
1.2. Method 1 or 1.1, wherein promoting resolution of inflammation comprises promoting activation of M2 macrophages.
1.3. Method 1 or 1.1 wherein the disease or condition to be treated is selected from bacterial infections selected from *Salmonella typhi, Salmonella typhimurium, Listeria monocytogenes, Mycobacterium tuberculosis, Mycobacterium ulcerans,* and *Mycobacterium avium* infections; viral infections selected from African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, Viral Hepatitis, which is Hepatitis A, Hepatitis B, or Hepatitis C; parasitic infestations, which are *Taenia crassiceps, Toxoplasma gondii, Leishmania infantum, Schistosoma mansoni* infestations; atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis.
1.4. Any foregoing method wherein the disease or condition to be treated is a bacterial infection.
1.5. Any foregoing method wherein the disease or condition to be treated is a *Salmonella typhi, Salmonella typhimurium, Listeria monocytogenes, Mycobacterium tuberculosis, Mycobacterium ulcerans,* or *Mycobacterium avium* infection.
1.6. Method 1-1.3, wherein the disease or condition to be treated is a viral infection.
1.7. Method 1.6, wherein the viral infection is African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, or Viral Hepatitis, which is Hepatitis A, Hepatitis B, or Hepatitis C.
1.8. Method 1-1.3, wherein the disease or condition to be treated is a parasitic infestation.
1.9. Method 1.8, wherein the parasitic infestation is a *Taenia crassiceps, Toxoplasma gondii, Leishmania infantum,* or *Schistosoma mansoni* infestation.
1.10. Method 1-1.3, wherein the disease or condition to be treated is atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis.
1.11. Method 1-1.3 or 1.10, wherein the disease or condition to be treated is nonalcoholic steatoheptatitis (NASH); autoimmune hepatitis; systemic lupus erythematosus (SLE); wound healing; pleurisy; peritonitis; and cystic fibrosis.
1.12. Any foregoing method comprising administering an effective amount of a PDE1 inhibitor of the current invention (e.g., a PDE1 inhibitor of Formulas I, Ia, II, III, and/or IV as herein described) in an amount effective to (i) reduce or inhibit activation of M1 macrophages, and/or (ii) an amount effective to reduce levels of one or more pro-inflammatory cytokines (e.g., IL1β, TNF- α, IL6 and Ccl2, or combination thereof); to a patient in need thereof.
1.13. Any foregoing method comprising administering an effective amount of a PDE1 inhibitor of the current invention (e.g., a PDE1 inhibitor of Formulas I, Ia, II, III, and/or IV as herein described) to a patient in need thereof, in an amount effective to (i) promote activation of M2 macrophages, and/or (ii) an amount effective to promote anti-inflammatory cytokines (e.g., IL-10).
1.14. Any foregoing method comprising administering an effective amount of a PDE1 inhibitor of the current invention (e.g., a PDE1 inhibitor of Formulas I, Ia, II, III, and/or IV as herein described) to a patient in need thereof, in an amount effective to reduce levels of macrophages of the M1 phenotype and/or enhance levels of macrophages of the M2 phenotype.
1.15. Any foregoing method wherein the inflammation is associated with increased expression and/or activation of macrophages (e.g., M1 macrophages).
1.16. Any foregoing method wherein the PDE1 inhibitor blunts or inhibits the expression and/or activity of pro-inflammatory cytokines, e.g., selected from the group consisting of: IL1B, IL-6, TNF- α, Ccl2, Nitric Oxide (NO), and Reactive Oxygen Species (ROS).
1.17. Any foregoing method wherein the PDE1 inhibitor in administered in combination with a PDE4 inhibitor (e.g., rolipram).
1.18. Any foregoing method wherein the patient exhibits increased levels of pro-inflammatory cytokines (e.g., IL1B, IL6, TNF-alpha, Ccl2).
1.19. Any foregoing method wherein "PDE1 inhibitor" describes a compound(s) which selectively inhibit phosphodiesterase-mediated (e.g., PDE1-mediated, especially PDE1B-mediated) hydrolysis of cGMP, e.g., with an IC₅₀ of less than 1µM, preferably less than 750 nM, more preferably less than 500 nM, more preferably less than 50 nM in an immobilized-metal affinity particle reagent PDE assay.
1.20. Any foregoing method wherein the PDE1 inhibitor inhibits the activity of PDE1 (e.g., bovine PDE1 in the assay described in Example 1) with an IC₅₀ of less than 10 nM, e.g., wherein the PDE1 inhibitor does not inhibit the activity of PDE types other than PDE1, e.g., has an IC₅₀ at least 1000 times greater for PDE types other than PDE1.
1.21. Any foregoing method, wherein the PDE1 inhibitor is the following: 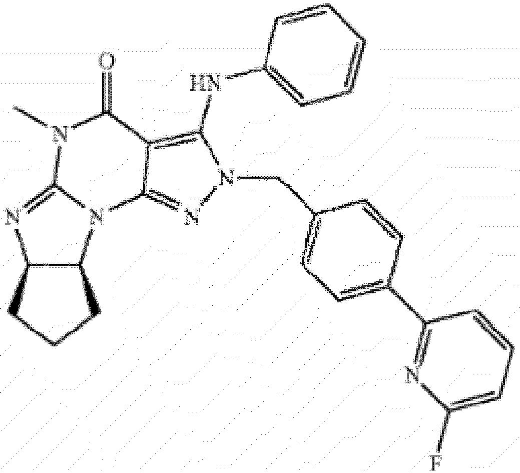 in free or pharmaceutically acceptable salt form.
1.22. Any foregoing method, wherein the PDE1 inhibitor is the following: 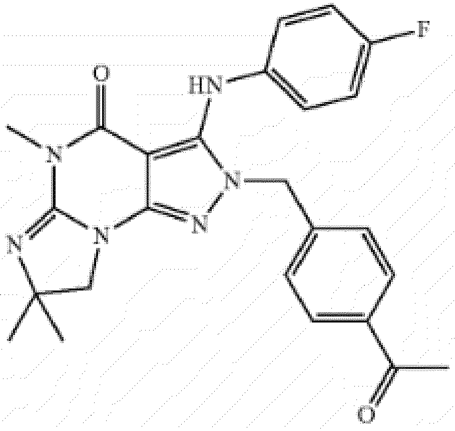 in free or pharmaceutically acceptable salt form.
1.23. Any foregoing method, wherein the PDE1 inhibitor is the following: 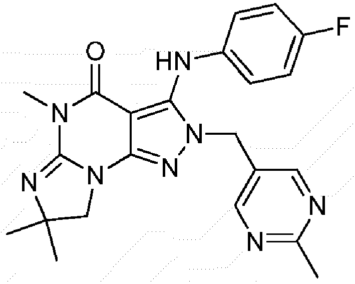 in free or pharmaceutically acceptable salt form.
1.24. Any foregoing method, wherein the PDE1 inhibitor is the following: 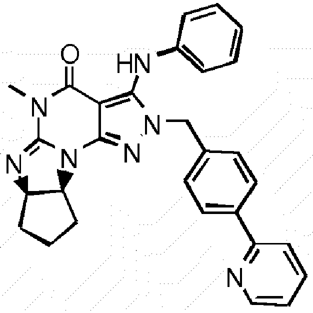 in free or pharmaceutically acceptable salt form.
1.25. Any of the foregoing method wherein the patient has elevated levels of one or more pro-inflammatory cytokines (e.g., selected from IL1β, TNFα, Ccl2, IL-6, and combinations thereof).
1.26. Any of the foregoing method wherein the patient has reduced levels of one or more anti-inflammatory cytokines (e.g., IL-10).
1.27. Any of the foregoing method wherein the patient has elevated levels of macrophages of the M1 phenotype compared to macrophages of the M2 phenotype.
1.28. Any of the foregoing methods wherein the patient is also administered one or more of an antibiotic agent, antiviral agent, corticosteroids or NSAIDs.

For example, in exemplary embodiments of claim **1,** the invention provides a method (Method 2) of promoting macrophage activation to the M2 activation state, the method comprising administering an effective amount of a specific inhibitor of phosphodiesterase type I (PDE1), to a patient suffering from inflammation or a disease or condition associated with inflammation (e.g., macrophage-mediated inflammation), for example:
2.1 Method 2, wherein the disease or condition to be treated is selected from bacterial infections (e.g., *Salmonella typhi, Salmonella typhimurium, Listeria monocytogenes, Mycobacterium tuberculosis, Mycobacterium ulcerans,* and *Mycobacterium avium* infections); viral infections (e.g., African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, Viral Hepatitis (e.g., Hepatitis A, Hepatitis B, Hepatitis C)); parasitic infestations (e.g., *Taenia crassiceps, Toxoplasma gondii, Leishmania infantum, Schistosoma mansoni* infestations); atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis.
2.2 Any foregoing method wherein the disease or condition to be treated is a bacterial infection.
2.3 Any foregoing method wherein the disease or condition to be treated is a *Salmonella typhi, Salmonella typhimurium, Listeria monocytogenes, Mycobacterium tuberculosis, Mycobacterium ulcerans,* or *Mycobacterium avium* infection.
2.4 Method 2 or 2.1, wherein the disease or condition to be treated is a viral infection.
2.5 Method 2 or 2.4, wherein the viral infection is African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, or Viral Hepatitis (e.g., Hepatitis A, Hepatitis B, Hepatitis C).
2.6 Method 2 or 2.1, wherein the disease or condition to be treated is a parasitic infestation.
2.7 Method 2.7, wherein the parasitic infestation is a *Taenia crassiceps, Toxoplasma gondii, Leishmania infantum,* or *Schistosoma mansoni* infestation.
2.8 Method 2 or 2.1, wherein the disease or condition to be treated is atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis.
2.9 Method 2 or 2.8, wherein the disease or condition to be treated is nonalcoholic steatoheptatitis (NASH); autoimmune hepatitis; systemic lupus erythematosus (SLE); wound healing; pleurisy; peritonitis; and cystic fibrosis.
2.10 Any foregoing method comprising administering an effective amount of a PDE1 inhibitor of the current invention (e.g., a PDE1 inhibitor of Formulas I, Ia, II, III, and/or IV as herein described) in an amount effective to (i) reduce or inhibit activation of M1 macrophages, and/or (ii) an amount effective to reduce levels of one or more pro-inflammatory cytokines (e.g., IL1β, TNF- α, IL6 and Ccl2, or combination thereof); to a patient in need thereof.
2.11 Any foregoing method comprising administering an effective amount of a PDE1 inhibitor of the current invention (e.g., a PDE1 inhibitor of Formulas I, Ia, II, III, and/or IV as herein described) to a patient in need thereof, in an amount effective to (i) promote activation of M2 macrophages, and/or (ii) an amount effective to promote anti-inflammatory cytokines (e.g., IL-10).
2.12 Any foregoing method comprising administering an effective amount of a PDE1 inhibitor of the current invention (e.g., a PDE1 inhibitor of Formulas I, Ia, II, III, and/or IV as herein described) to a patient in need thereof, in an amount effective to reduce levels of macrophages of the M1 phenotype and/or enhance levels of macrophages of the M2 phenotype.
2.13 Any foregoing method wherein the inflammation is associated with increased expression and/or activation of macrophages (e.g., M1 macrophages).
2.14 Any foregoing method wherein the PDE1 inhibitor blunts or inhibits the expression and/or activity of pro-inflammatory cytokines, e.g., selected from the group consisting of: IL1B, IL-6, TNF- α, Ccl2, Nitric Oxide (NO), and Reactive Oxygen Species (ROS).
2.15 Any foregoing method wherein the PDE1 inhibitor in administered in combination with a PDE4 inhibitor (e.g., rolipram).
2.16 Any foregoing method wherein the patient exhibits increased levels of pro-inflammatory cytokines (e.g., IL1B, IL6, TNF-alpha, Ccl2).
2.17 Any foregoing method wherein "PDE1 inhibitor" describes a compound(s) which selectively inhibit phosphodiesterase-mediated (e.g., PDE1-mediated, especially PDE1B-mediated) hydrolysis of cGMP, e.g., with an IC₅₀ of less than 1µM, preferably less than 750 nM, more preferably less than 500 nM, more preferably less than 50 nM in an immobilized-metal affinity particle reagent PDE assay.
2.18 Any foregoing method wherein the PDE1 inhibitor inhibits the activity of PDE1 (e.g., bovine PDE1 in the assay described in Example 1) with an IC₅₀ of less than 10 nM, e.g., wherein the PDE1 inhibitor does not inhibit the activity of PDE types other than PDE1, e.g., has an IC₅₀ at least 1000 times greater for PDE types other than PDE1.
2.19 Any foregoing method, wherein the PDE1 inhibitor is the following: 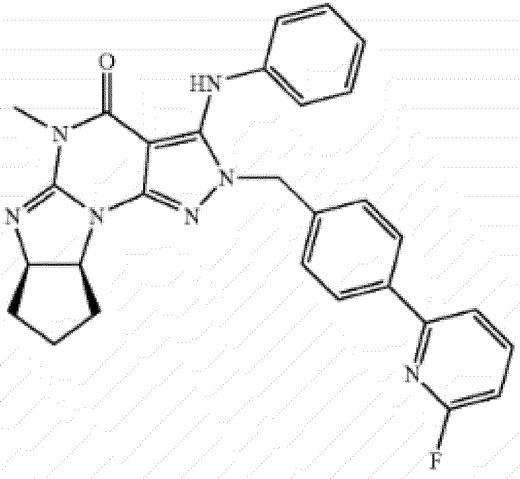 in free or pharmaceutically acceptable salt form.
2.20 Any foregoing method, wherein the PDE1 inhibitor is the following: 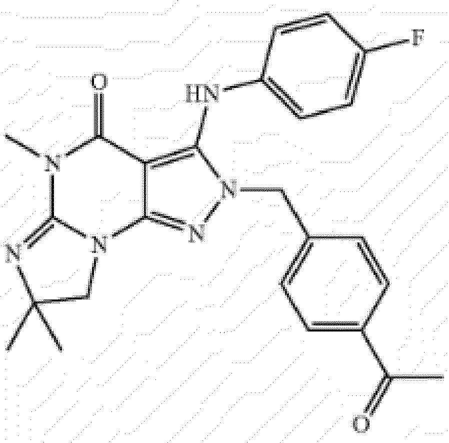 in free or pharmaceutically acceptable salt form.
2.21 Any foregoing method, wherein the PDE1 inhibitor is the following: 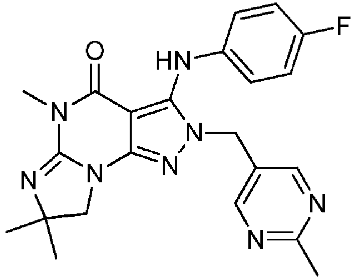 in free or pharmaceutically acceptable salt form.
2.22 Any foregoing method, wherein the PDE1 inhibitor is the following: 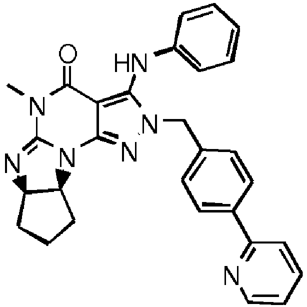 in free or pharmaceutically acceptable salt form.
2.23 Any of the foregoing method wherein the patient has elevated levels of one or more pro-inflammatory cytokines (e.g., selected from IL1β, TNFα, Ccl2, IL-6, and combinations thereof).
2.24 Any of the foregoing method wherein the patient has reduced levels of one or more anti-inflammatory cytokines (e.g., IL-10).
2.25 Any of the foregoing method wherein the patient has elevated levels of macrophages of the M1 phenotype compared to macrophages of the M2 phenotype.
2.26 Any of the foregoing methods wherein the patient is also administered one or more of an antibiotic agent, antiviral agent, corticosteroids or NSAIDs.

The invention further provides a PDE1 inhibitor, which is a Compound of Formulas I, Ia, II, III, and/or IV for use in any of Methods 1, et seq.

The invention further provides a pharmaceutical composition comprising a PDE1 inhibitor of Formulas I, Ia, II, III, and/or IV for use in any of Methods 1 et seq.

The phrase "Compounds of the Invention" or "PDE 1 inhibitors of the Invention", or like terms, encompasses any and all of the compounds disclosed herewith, i.e., a Compound of Formulas I, Ia, II, III, and/or IV.

The words "treatment" and "treating" are to be understood accordingly as embracing prophylaxis and treatment or amelioration of symptoms of disease as well as treatment of the cause of the disease.

For methods of treatment, the word "effective amount" is intended to encompass a therapeutically effective amount to treat or mitigate a specific disease or disorder, and/or a symptom thereof, and/or to reduce inflammatory cytokines, e.g., as produced by macrophages, and/or to reduce M1 macrophage activation, and/or to increase anti-inflammatory cytokines, e.g., as produced by macrophages, and/or to enhance M2 macrophage activation.

The term "patient" includes a human or non-human (i.e., animal) patient. In a particular embodiment, the invention encompasses both humans and nonhuman animals. In another embodiment, the invention encompasses nonhuman animals. In other embodiments, the term encompasses humans.

The term "comprising" as used in this disclosure is intended to be open-ended and does not exclude additional, unrecited elements or method steps.

Compounds of the Invention, i.e., Formulas I, Ia, II, III, and/or IV as hereinbefore described, in free or pharmaceutically acceptable salt form, may be used as a sole therapeutic agent, but may also be used in combination or for co-administration with other active agents.

For example, in certain embodiments, the Compounds of Formulas I, Ia, II, III, and/or IV as hereinbefore described, in free or pharmaceutically acceptable salt form, may be administered in combination (e.g. administered sequentially or simultaneously or within a 24 hour period) with other active agents, e.g., with one or more antidepressant agents, e.g., with one or more compounds in free or pharmaceutically acceptable salt form, selected from selective serotonin reuptake inhibitors (SSRIs), ) serotonin-norepinephrine reuptake inhibitors (SNRIs), c) tricyclic antidepressants (TCAs), and atypical antipsychotics.

Dosages employed in practicing the present invention will of course vary depending, e.g. on the particular disease or condition to be treated, the particular Compound of the Invention used, the mode of administration, and the therapy desired. Compounds of the Invention may be administered by any suitable route, including orally, parenterally, transdermally, or by inhalation, but are preferably administered orally. In general, satisfactory results, e.g. for the treatment of diseases as hereinbefore set forth are indicated to be obtained on oral administration at dosages of the order from about 0.01 to 2.0 mg/kg. In larger mammals, for example humans, an indicated daily dosage for oral administration will accordingly be in the range of from about 0.75 to 150 mg (depending on the drug to be administered and the condition to be treated, for example in the case of Compound 214, 0.5 to 25 mg, e.g., 1 to 10 mg, per diem, e.g., in monophosphate salt form, for treatment of inflammatory conditions), conveniently administered once, or in divided doses 2 to 4 times, daily or in sustained release form. Unit dosage forms for oral administration thus for example may comprise from about 0.2 to 75 or 150 mg, e.g. from about 0.2 or 2.0 to 50, 75 or 100 mg (e.g., 1, 2.5, 5, 10, or 20 mg) of a Compound of the Invention, e.g., together with a pharmaceutically acceptable diluent or carrier therefor.

Pharmaceutical compositions comprising Compounds of the Invention may be prepared using conventional diluents or excipients and techniques known in the galenic art. Thus oral dosage forms may include tablets, capsules, solutions, suspensions and the like.

### EXAMPLES

### Example 1: Peripheral Inflammation Assessment Using Mouse Zymosan Pleurisy Model

Zymosan is injected into the pleural cavities of mice in order to induce sterile inflammation. Infiltration of leukocytes, neutrophils, and macrophages are monitored at days 3 and 7 following injection. Detection of various cell types are identified according to the gating strategy outlined in Table 1 below.

**Table 1: Cell types and identifiable markers for flow cytometry**

| **Cell Type** | **Expressed Markers** |
|---|---|
| Leukocytes | CD45+ |
| Neutrophils | CD45+/Ly6G+ |
| Macrophages | CD45+/Ly6G-/CD19-/CD11c-/CD11b+/F4/80+ |
| M1 Macrophages | CD45+/Ly6G-/CD19-/CD11c-/CD11b+/F4/80+CD38+ |
| M2 Macrophages | CD45+/Ly6G-/CD19-/CD11c-/CD11b+/F4/80+/EGR2+ |

In this model, injection of zymosan causes the recruitment of various waves of leukocytes, which are observed and recorded. Exudate volume increases to a maximum over a period of 24 hours, and neutrophils increase within 4 hours and reach a maximum by 48 hours. Lymphocytes of the adaptive immune system enter at a later stage, after three days, which is signaled by macrophages presenting antigens. A resolution phase is well documented in this model and is accompanied by decreased total macrophage number and transition into M2 phenotype.

In the studies, Compounds 1 and 2 were administered to the test subjects, and the effect the compounds had on infiltration of leukocytes, neutrophils, and macrophages was observed.

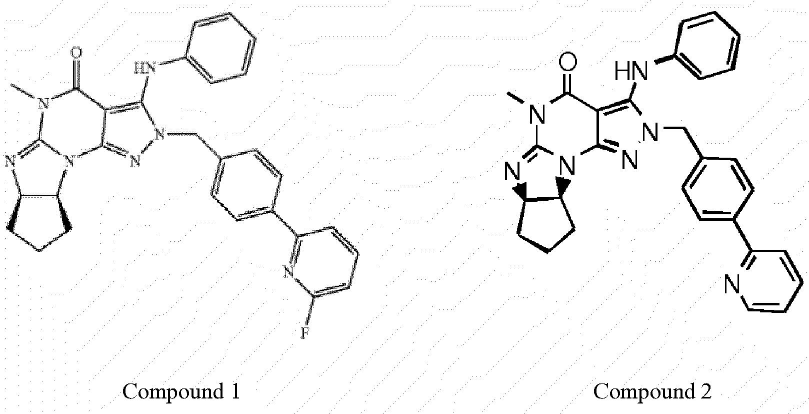

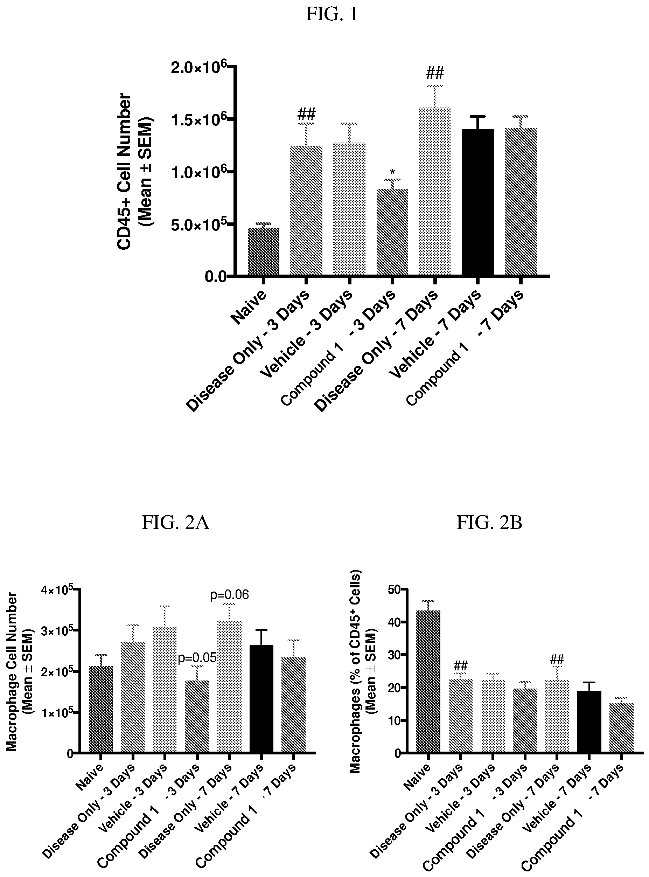

As shown in the accompanying figures 1-9, it was observed that the subjects treated with Compound 1 or 2 showed enhanced inflammatory resolution by promoting shift from M1 to M2. The data show that in the treated specimens, inflammation due to M1 macrophages was consistently decreased, while M2 activation was promoted. As shown in Figure **1,** 1 mg of Zymosan i.p. injection into the peritoneal cavity resulted in significant total CD45+ leukocyte infiltration. This increased total number of leukocytes resulted in a general increase in total macrophage numbers on day 3 and 7 following Zymosan injection in disease only animals compared to naive (Figure 2A). The percentage of the macrophages based on the total number of leukocytes (Figure 2B) slightly decreased between days 3 and 7.

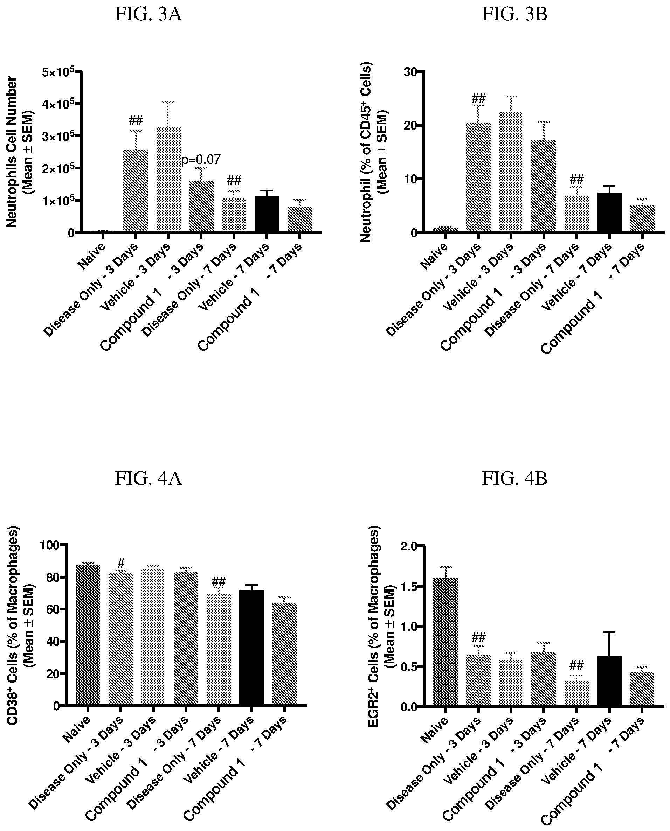

The number of neutrophils dropped significantly on day 7 in the disease only and vehicle tested animals, while the animals administered Compound 1 showed a less dramatic decrease (Figure 3A). These results are reflected in Figure 3B, which showed that the overall percentage of neutrophils relative to CD45+ leukocytes dropped significantly for all subjects.

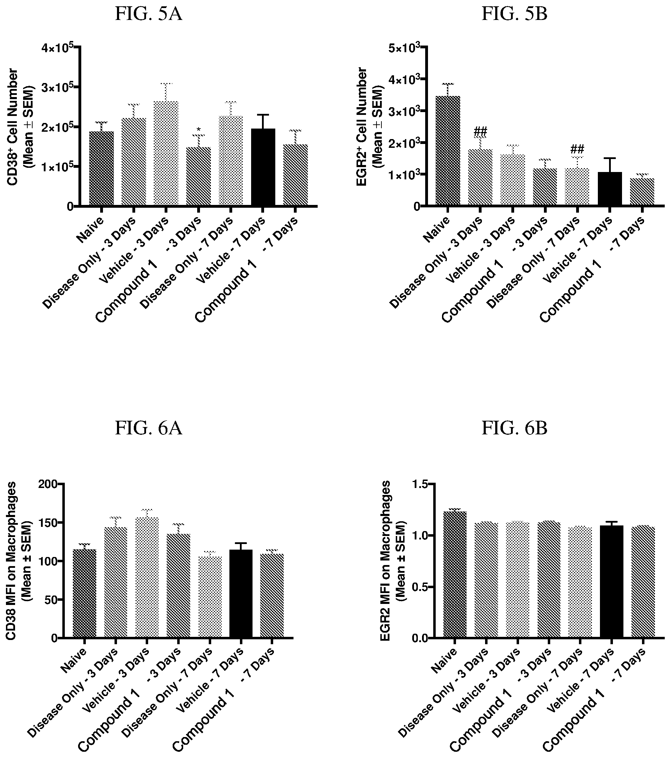

To further assess the CD38 and Egr2 expression on macrophages, total numbers of CD38+ macrophages and Egr2+ macrophages were analyzed. Total numbers of CD38+ macrophages were increased in disease and vehicle day 3 animal groups, but decreased on day 3 for animals treated with Compound 1 (Figure 5A). The number of Egr2+ macrophages was decreased for all animal groups at day 3 (Figure 5B). The mean fluorescence intensity (MFI) of both CD38 and Egr2 was also analyzed on macrophages in Figures 6A and 6B. MFI provides a number that relates to the relative expression of a given marker on a cellular population. MFI for CD38+ showed an increase on day 3 for all animal groups, with the lowest value for the group treated with Compound 1, and decreased on day 7 for all groups. On the other hand, the MFI for Egr2+ was decreased on all animal groups on days 3 and 7, when compared to naive.

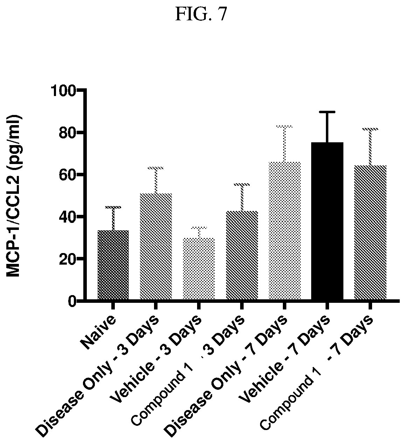

Overall, the results indicate that the number of CD38+ cells tended to decrease and the number of Egr2+ cell number and percent tended to increase indicating a trend to increase the resolution phase of the inflammatory insult on day 7. As shown in Figure 7, animals treated with Compound 1 also tended to show less inflammatory biomarkers (MCP-1/CCL2) at 3 and 7 days in comparison with control groups.

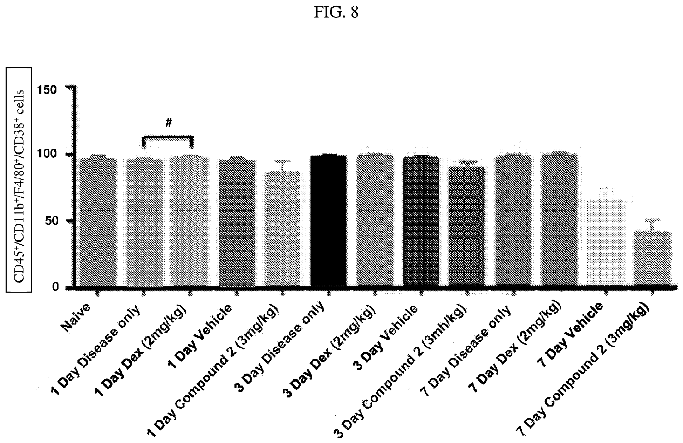

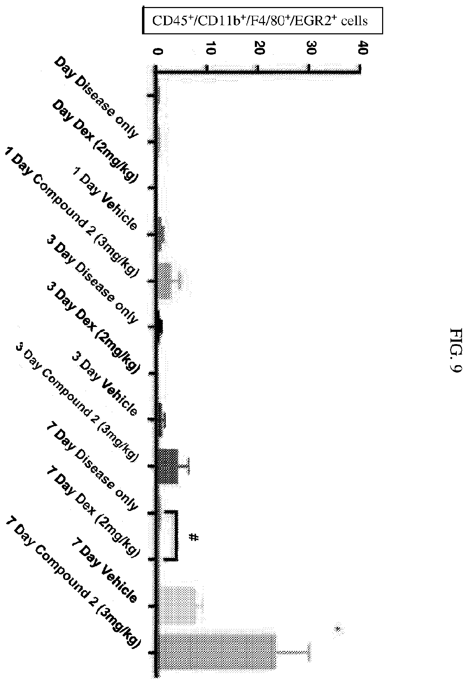

Similar tests were conducted with Compound 2, the results of which are illustrated in Figures 8 and 9. Treatment with 2 mg/kg of Dexamethasone had no significant effect on the number of CD38+ or Erg2+ macrophage populations on day 3 or 7. Treatment with 3 mg/kg of Compound 2, however, resulted in a significant drop of CD38+ macrophages, which corresponded with a sharp and significant increase in Erg2+ macrophages on day 7.An additional test was carried out according to the same method. Mice were injected intraperitoneally with 1mg Zymosan followed by 10mg/kg Compound 1. Macrophage levels were recorded at injection, then at 4, 8, 16, 24, 48 and 72 hours post-injection. The results are summarized in Figures 11A, 11B, 12A and 12B. As shown in Figures 11A and 11B, treatment with Compound 1 resulted in lower M1 macrophage levels at all observed times, with a significant different observed at day 7 in CD80+ macrophages. Correspondingly, Arg1+ M2 macrophages increased at 4 hours, and CD206+ M2 macrophages significantly increased at relative to control at 2 and 3 days.

### Example 2: Effect of PDE1 Inhibitor on Microglia Chemotaxis Assay (reference example)

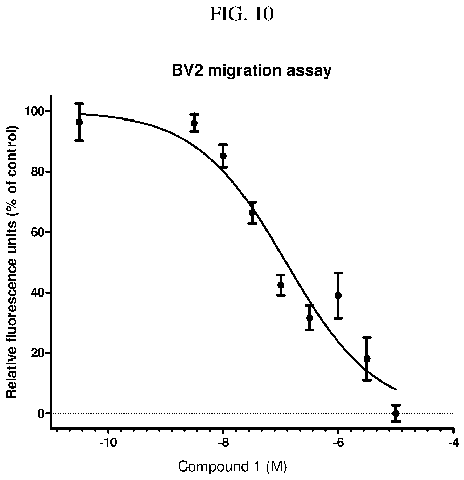

BV2 cells were added to upper chamber of a 5 µm pore Transwell 96-well plate over a reservoir containing 100 µM ADP and incubated at 37°C with 5% CO2 for 4 hours. After the incubation cells were harvested with pre-warmed cell detachment solution for 30 minutes in the same incubation conditions. 75 µl of this cell detachment solution was combined with 75 µl of culture medium in a new 96 well plate compatible with a fluorescence reader. Cell number in bottom chamber was determined by adding CyQuant^{®} GR dye and reading in the Envision fluorescence reader at 480 nm EX /520 nm EM. CyQuant^{®} GR dye exhibits strong fluorescence when bound to nucleic acid and is accurate enough to measure differences down to single cells. As shown in Figure 10, the presence of the PDE1 inhibitor Compound 1 showed a marked dampening effect on the motility of the BV2 cells across the membrane, providing additional evidence that Compound 1 dampens the release of pro-inflammatory markers.

### Example 3: Detection of Inflammatory Biomarkers Using Mouse Zymosan Pleurisy Model

Zymosan was injected into the pleural cavities of mice in order to induce sterile inflammation by the methods discussed in Example 1. Compound 1 was administered to test subjects to observe the effects on a variety of inflammatory biomarkers. Results were recorded after 4 hours. The subjects showed a clear decrease in cytokine markers following administration of Compound 1. IFNγ, IL-1β, MCP1-β and TNF-α decreased following administration of Compound 1 in all serum and plasma samples. IL10 showed a decrease in serum.

Lipids are known to be involved in regulation of a multitude of cellular responses including cell growth and death, and inflammation/infection, via receptor-mediated pathways. Various lipids are involved in both the initiation and resolution of inflammation. Pro-resolving lipid mediators are produced naturally in the body from unsaturated fatty acids, such as arachidonic acid (AA) and docosahexaenoic acid (DHA). Further studies were carried out to identify metabolites of AA and DHA, which are summarized below in Tables 2 and 3.

**Table 2: Detection of Arachidonic Acid Metabolites**

| **Metabolite** | **Inflammation Function** | **Result** |
|---|---|---|
| TXB2 | Pro-inflammatory mediator | Decrease |
| PGE2 | Pro-inflammatory mediator | Decrease |
| LTB4 | Pro-inflammatory mediator | No change |
| 5-HETE | Intermediate mediator linked to resolution | No change |
| 12-HETE | Intermediate mediator linked to resolution | Increase |
| 15-HETE | Intermediate mediator linked to resolution | Increase |

**Table 3: Detection of Docosahexaenoic Acid Metabolites**

| **Metabolite** | **Inflammation Function** | **Result** |
|---|---|---|
| 17-HDOHE | Intermediate mediator linked to resolution | Increase |
| RVD5 | Intermediate mediator linked to resolution | Increase |
| 14-HDOHE | Resolution mediator | Increase |

As shown above in relation to AA metabolism, 12-HETE and 15-HETE, both intermediate mediators leading to resolution of inflammation, show increased occurrence compared with controls, while pro-inflammatory mediators TXB2, PGE2 and LTB4 all decrease. For the metabolism of DHA, each of 17-HDOHE, RVD5 and 14-HDOHE increase, all of which are related to resolution of inflammation. This profile of lipid biomarkers suggests that the tested compound induces metabolites of 15-LOX and 12-LOX pathways, indicating a mobilization of pro-resolution pathways. It also shows that the tested compound does not induce metabolites of 5-LOX, which is a pro-inflammatory pathway.

## Claims

1. A PDE1 inhibitor or a pharmaceutical composition comprising a PDE1 inhibitor for use in a method of promoting resolution of inflammation for the treatment or prophylaxis of inflammation or disease associated with inflammation, the method comprising administering the PDE1 inhibitor or pharmaceutical composition to a patient in need thereof, wherein the patient is suffering from a disease or disorder mediated by macrophages, selected from bacterial infections selected from *Salmonella typhi, Salmonella typhimurium, Listeria monocytogenes, Mycobacterium tuberculosis, Mycobacterium ulcerans,* and *Mycobacterium avium* infections; viral infections selected from African Swine Fever Virus, Classical Swine Fever Virus, Dengue Virus, Foot and Mouth Disease Virus, Influenza A Virus, Porcine Circovirus-2, Porcine Reproductive and Respiratory Syndrome Virus, Porcine Pseudorabies Virus, Respiratory Syncytial Virus, Severe Acute Respiratory Syndrome Coronavirus, West Nile Virus, Viral Hepatitis (e.g., Hepatitis A, Hepatitis B, Hepatitis C); parasitic infestations (e.g., *Taenia crassiceps, Toxoplasma gondii, Leishmania infantum, Schistosoma mansoni* infestations); atopic dermatitis; pneumonia; obesity and insulin resistance; asthma; pulmonary fibrosis; chronic obstructive pulmonary disease (COPD); diabetes; sepsis; nonalcoholic steatoheptatitis (NASH); autoimmune hepatitis; systemic lupus erythematosus (SLE); wound healing; pleurisy; peritonitis; and cystic fibrosis, and
wherein the PDE1 inhibitor is a compound selected from
**(A) Formula I:** 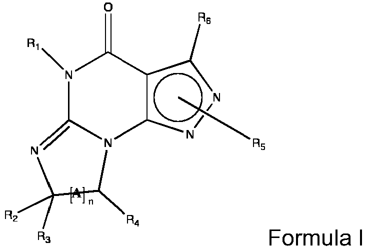 wherein
(i) R₁ is H or C₁₋₄ alkyl (e.g., methyl);
(ii) R₄ is H or C₁₋₄ alkyl and R₂ and R₃ are, independently, H or C₁₋₄ alkyl (e.g., R₂ and R₃ are both methyl, or R₂ is H and R₃ is isopropyl), aryl, heteroaryl, (optionally hetero)arylalkoxy, or (optionally hetero)arylalkyl; or
R₂ is H and R₃ and R₄ together form a di-, tri- or tetramethylene bridge (pref. wherein the R₃ and R₄ together have the *cis* configuration, e.g., where the carbons carrying R₃ and R₄ have the R and S configurations, respectively);
(iii) R₅ is a substituted heteroarylalkyl, e.g., substituted with haloalkyl;
or R₅ is attached to one of the nitrogens on the pyrazolo portion of Formula I and is a moiety of Formula A 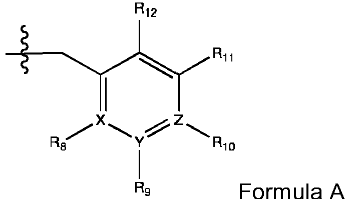 wherein X, Y and Z are, independently, N or C, and R₈, R₉, R₁₁ and R₁₂ are independently H or halogen (e.g., Cl or F), and R₁₀ is halogen, alkyl, cycloalkyl, haloalkyl (e.g., trifluoromethyl), aryl (e.g., phenyl), heteroaryl (e.g., pyridyl (for example pyrid-2-yl) optionally substituted with halogen, or thiadiazolyl (e.g., 1,2,3-thiadiazol-4-yl)), diazolyl, triazolyl, tetrazolyl, arylcarbonyl (e.g., benzoyl), alkylsulfonyl (e.g., methylsulfonyl), heteroarylcarbonyl, or alkoxycarbonyl; provided that when X, Y, or Z is nitrogen, R₈, R₉, or R₁₀, respectively, is not present; and
(iv) R₆ is H, alkyl, aryl, heteroaryl, arylalkyl (e.g., benzyl), arylamino (e.g., phenylamino), heteroarylamino, N,N-dialkylamino, N,N-diarylamino, or N-aryl-N-(arylalkyl)amino (e.g., N-phenyl-N-(1,1'-biphen-4-ylmethyl)amino); and
(v) n=0 or 1;
(vi) when n=1, A is -C(R₁₃R₁₄)-
wherein R₁₃ and R₁₄, are, independently, H or C₁₋₄ alkyl, aryl, heteroaryl, (optionally hetero)arylalkoxy or (optionally hetero)arylalkyl;
in free or salt form, including its enantiomers, diastereoisomers and racemates;
**(B) Formula Ia:** 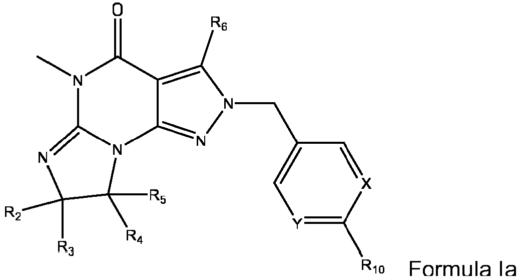 wherein
(i) R₂ and R₅ are independently H or hydroxy and R₃ and R₄ together form a tri- or tetra-methylene bridge [pref. with the carbons carrying R₃ and R₄ having the R and S configuration respectively]; or R₂ and R₃ are each methyl and R₄ and R₅ are each H; or R₂, R₄ and R₅ are H and R₃ is isopropyl [pref. the carbon carrying R₃ having the R configuration];
(ii) R₆ is (optionally halo- or hydroxy-substituted) phenylamino, (optionally halo- or hydroxy-substituted) benzylamino, C₁₋₄alkyl, or C₁₋₄alkyl sulfide; for example, phenylamino or 4-fluorophenylamino;
(iii) R₁₀ is C₁₋₄alkyl, methylcarbonyl, hydroxyethyl, carboxylic acid, sulfonamide, (optionally halo- or hydroxy-substituted) phenyl, (optionally halo- or hydroxy-substituted) pyridyl (for example 6-fluoropyrid-2-yl), or thiadiazolyl (e.g., 1,2,3-thiadiazol-4-yl); and
(iv) X and Y are independently C or N,
in free or pharmaceutically acceptable salt form, including its enantiomers, diastereoisomers and racemates;
**(C) Formula II:** 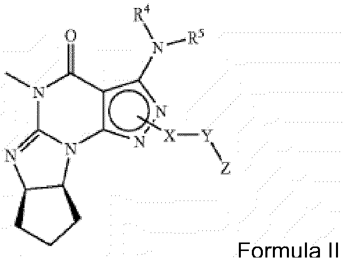
(i) X is C₁₋₆alkylene (e.g., methylene, ethylene or prop-2-yn-1-ylene);
(ii) Y is a single bond, alkynylene (e.g., -C≡C-), arylene (e.g., phenylene) or heteroarylene (e.g., pyridylene);
(iii) Z is H, aryl (e.g., phenyl), heteroaryl (e.g., pyridyl, e.g., pyrid-2-yl), halo (e.g., F, Br, Cl), haloC₁₋₆alkyl (e.g., trifluoromethyl), -C(O)-R¹, -N(R²)(R³), or C₃₋₇cycloalkyl optionally containing at least one atom selected from a group consisting of N or O (e.g., cyclopentyl, cyclohexyl, tetrahydro-2H-pyran-4-yl, or morpholinyl);
(iv) R¹ is C₁₋₆alkyl, haloC₁₋₆alkyl, -OH or -OC₁₋₆alkyl (e.g., -OCH₃);
(v) R² and R³ are independently H or C₁₋₆alkyl;
(vi) R⁴ and R⁵ are independently H, C₁₋₆alky or aryl (e.g., phenyl) optionally substituted with one or more halo (e.g., fluorophenyl, e.g., 4-fluorophenyl), hydroxy (e.g., hydroxyphenyl, e.g., 4-hydroxyphenyl or 2-hydroxyphenyl) or C₁₋₆alkoxy;
(vii) wherein X, Y and Z are independently and optionally substituted with one or more halo (e.g., F, Cl or Br), C₁₋₆alkyl (e.g., methyl), haloC₁₋₆alkyl (e.g., trifluoromethyl), for example, Z is heteroaryl, e.g., pyridyl substituted with one or more halo (e.g., 6-fluoropyrid-2-yl, 5-fluoropyrid-2-yl, 6-fluoropyrid-2-yl, 3-fluoropyrid-2-yl, 4-fluoropyrid-2-yl, 4,6-dichloropyrid-2-yl), haloC₁₋₆alkyl (e.g., 5-trifluoromethylpyrid-2-yl) or C₁₋₆-alkyl (e.g., 5-methylpyrid-2-yl), or Z is aryl, e.g., phenyl, substituted with one or more halo (e.g., 4-fluorophenyl),
in free or salt form;
**(D) Formula III:** 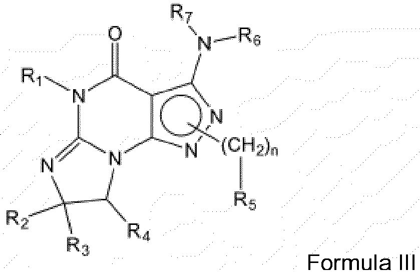 wherein
(i) R1 is H or C₁₋₄ alkyl (e.g., methyl or ethyl);
(ii) R₂ and R₃ are independently H or C₁₋₆ alkyl (e.g., methyl or ethyl);
(iii) R₄ is H or C₁₋₄ alkyl (e.g., methyl or ethyl);
(iv) R₅ is aryl (e.g., phenyl) optionally substituted with one or more groups independently selected from -C(=O)-C₁₋₆ alkyl (e.g., -C(=O)-CH₃) and C₁₋₆ - hydroxyalkyl (e.g., 1-hydroxyethyl);
(v) R₆ and R₇ are independently H or aryl (e.g., phenyl) optionally substituted with one or more groups independently selected from C₁₋₆ alkyl (e.g., methyl or ethyl) and halogen (e.g., F or CI), for example unsubstituted phenyl or phenyl substituted with one or more halogen (e.g., F) or phenyl substituted with one or more C₁₋₆ alkyl and one or more halogen or phenyl substituted with one C₁₋₆ alkyl and one halogen, for example 4-fluorophenyl or 3,4-difluorophenyl or 4-fluoro-3-methylphenyl; and
(vi) n is 1, 2, 3, or 4,
in free or salt form; and
**(E) Formula IV** 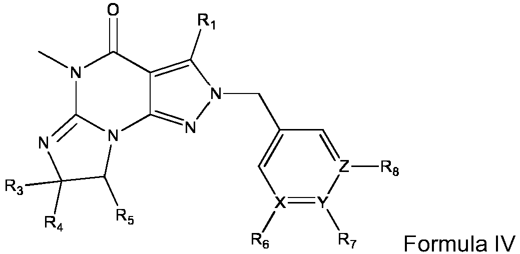 wherein
(i) R₁ is C₁₋₄alkyl (e.g., methyl or ethyl), or -NH(R₂), wherein R₂ is phenyl optionally substituted with halo (e.g., fluoro), for example, 4-fluorophenyl;
(ii) X, Y and Z are, independently, N or C;
(iii) R₃, R₄ and R₅ are independently H or C₁₋₄alkyl (e.g., methyl); or R₃ is H and R₄ and R₅ together form a tri-methylene bridge (pref. wherein the R₄ and R₅ together have the cis configuration, e.g., where the carbons carrying R₄ and R₅ have the R and S configurations, respectively),
(iv) R₆, R₇ and R₈ are independently:
H,
C₁₋₄alkyl (e.g., methyl),
pyrid-2-yl substituted with hydroxy, or
-S(O)₂-NH₂;
(v) Provided that when X, Y and/or Z are N, then R₆, R₇ and/or R₈, respectively, are not present; and when X, Y and Z are all C, then at least one of R₆, R₇ or R₈ is - S(O)₂-NH₂ or pyrid-2-yl substituted with hydroxy,
in free or salt form.

2. The inhibitor or composition for the use of claim 1 wherein the patient has
a. elevated levels of one or more pro-inflammatory cytokines (e.g., selected from IL1β, TNFα, Ccl2, IL-6, and combinations thereof);
b. reduced levels of one or more anti-inflammatory cytokines (e.g., IL-10);
c. elevated levels of macrophages of the M1 phenotype compared to macrophages of the M2 phenotype.

3. The inhibitor or composition for the use of claim 1 or claim 2, wherein the PDE1 inhibitor is the following: 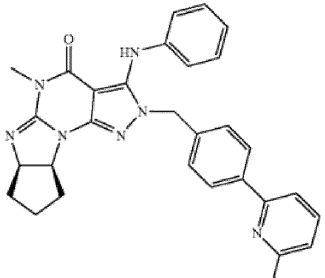 in free or pharmaceutically acceptable salt form.

4. The inhibitor or composition for the use of any preceding claim, wherein the PDE1 inhibitor is the following: 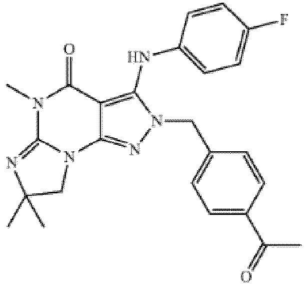 in free or pharmaceutically acceptable salt form.

5. The inhibitor or composition for the use of any preceding claim, wherein the PDE1 inhibitor is the following: 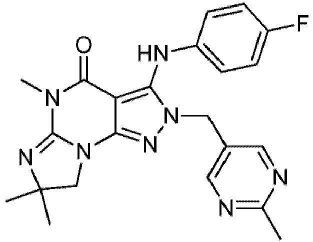 in free or pharmaceutically acceptable salt form.

6. The inhibitor or composition for the use of any preceding claim, wherein the PDE1 inhibitor is the following: 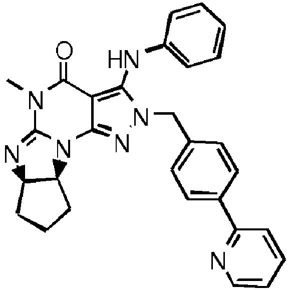 in free or pharmaceutically acceptable salt form.

7. The inhibitor or composition for the use of any of the preceding claims, wherein the PDE1 inhibitor is administered in combination with a PDE4 inhibitor (e.g., rolipram).

## Patentansprüche

1. PDE1-Inhibitor oder pharmazeutische Zusammensetzung, die einen PDE1-Inhibitor umfasst, zur Verwendung in einem Verfahren zur Förderung der Entzündungsauflösung zur Behandlung oder Prophylaxe von Entzündungen oder mit Entzündungen verbundenen Erkrankungen, wobei das Verfahren die Verabreichung des PDE1-Inhibitors oder der pharmazeutischen Zusammensetzung an einen Patienten umfasst, der diese benötigt, wobei der Patient an einer durch Makrophagen vermittelten Krankheit oder Störung leidet, ausgewählt aus bakteriellen Infektionen, ausgewählt aus Infektionen von *Salmonella typhi, Salmonella typhimurium, Listeria monocytogenes, Mycobacterium tuberculosis, Mycobacterium ulcerans* und *Mycobacterium avium;* Virusinfektionen, ausgewählt aus Afrikanischem Schweinepestvirus, Klassischem Schweinepestvirus, Dengue-Virus, Maul- und Klauenseuchevirus, Influenza-A-Virus, Porcinem Circovirus-2, Porcinem Reproduktions- und Respirationssyndromvirus, Porcinem Pseudorabiesvirus, Respiratorischem Synzytialvirus, Schwerem Akutem Respiratorischem Syndrom Coronavirus, West-Nil-Virus, Virushepatitis (z. B. Hepatitis A, Hepatitis B, Hepatitis C); Parasitenbefall (z. B. Befall von *Taenia crassiceps, Toxoplasma gondii, Leishmania infantum, Schistosoma mansoni);* atopischer Dermatitis; Pneumonie; Adipositas und Insulinresistenz; Asthma; Lungenfibrose; chronisch obstruktiver Lungenerkrankung (COPD); Diabetes; Sepsis; nichtalkoholischer Steatohepatitis (NASH); Autoimmunhepatitis; systemischem Lupus erythematodes (SLE); Wundheilung; Rippenfellentzündung; Bauchfellentzündung; und Mukoviszidose, und
wobei der PDE1-Inhibitor eine Verbindung ist, die ausgewählt ist aus
**(A) Formel I:** 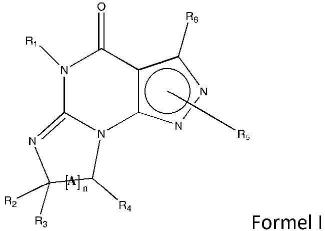 wobei
(i) R₁ H oder C₁₋₄-Alkyl (z.B. Methyl) ist;
(ii) R₄ H oder C₁₋₄-Alkyl ist und R₂ und R₃ unabhängig voneinander H oder C₁₋₄-Alkyl (z. B. sind R₂ und R₃ beide Methyl, oder R₂ ist H und R₃ ist Isopropyl), Aryl, Heteroaryl, (optional Hetero)arylalkoxy oder (optional Hetero)arylalkyl sind; oder
R₂ H ist und R₃ und R₄ zusammen eine Di-, Tri- oder Tetramethylenbrücke bilden (wobei vorzugsweise R₃ und R₄ zusammen die cis-Konfiguration aufweisen, z. B. wenn die kohlenstoffhaltigen R₃ und R₄ die R- bzw. S-Konfiguration aufweisen);
(iii) R₅ ein substituiertes Heteroarylalkyl ist, z.B. substituiert mit Halogenalkyl;
oder R₅ an eines der Stickstoffatome des Pyrazol-Teils der Formel I gebunden ist und eine Einheit der Formel A ist 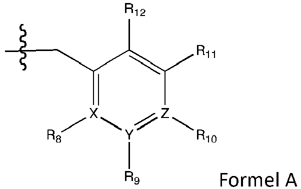
wobei X, Y und Z unabhängig voneinander N oder C sind und R₈, R₉, R₁₁ und R₁₂ unabhängig voneinander H oder Halogen (z. B. Cl oder F) sind und R₁₀ Halogen, Alkyl, Cycloalkyl, Halogenalkyl (z. B. Trifluormethyl), Aryl (z. B. Phenyl), Heteroaryl (beispielsweise Pyridyl (z. B. Pyrid-2-yl), optional mit Halogen substituiert, oder Thiadiazolyl (z. B. 1,2,3-Thiadiazol-4-yl)), Diazolyl, Triazolyl, Tetrazolyl, Arylcarbonyl (z. B. Benzoyl), Alkylsulfonyl (z. B. Methylsulfonyl), Heteroarylcarbonyl oder Alkoxycarbonyl ist; vorausgesetzt, dass R₈, R₉ bzw. R₁₀ nicht vorhanden ist, wenn X, Y oder Z Stickstoff ist; und
(iv) R₆ H, Alkyl, Aryl, Heteroaryl, Arylalkyl (z. B. Benzyl), Arylamino (z. B. Phenylamino), Heteroarylamino, N,N-Dialkylamino, N,N-Diarylamino oder N-Aryl-N-(arylalkyl)amino (z. B. N-Phenyl-N-(1,1'-biphen-4-ylmethyl)amino) ist; und
(v) n=0 oder 1;
(vi) wenn n=1, **A** -C(R₁₃R₁₄)- ist
wobei R₁₃ und R₁₄ unabhängig voneinander H oder C₁₋₄-Alkyl, Aryl, Heteroaryl, (optional Hetero)arylalkoxy oder (optional Hetero)arylalkyl sind;
in freier oder Salzform, einschließlich seiner Enantiomere, Diastereomere und Racemate;
**(B) Formel Ia:** 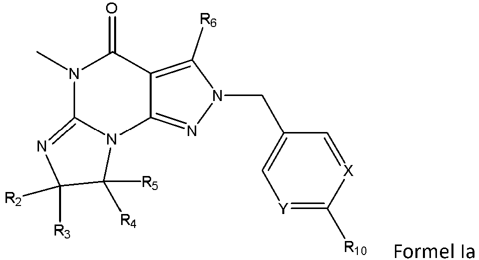 wobei
(i) R₂ und R₅ unabhängig voneinander H oder Hydroxy sind, und R₃ und R₄ zusammen eine Tri- oder Tetramethylenbrücke bilden [wobei vorzugsweise die kohlenstoffhaltigen R₃ und R₄ die R-bzw. S-Konfiguration aufweisen]; oder R₂ und R₃ jeweils Methyl sind und R₄ und R₅ jeweils H sind; oder R₂, R₄ und R₅ H sind und R₃ Isopropyl ist [wobei vorzugsweise das kohlenstoffhaltige R₃ die R-Konfiguration aufweist];
(ii) R₆ (optional halogen- oder hydroxy-substituiertes) Phenylamino, (optional halogen- oder hydroxy-substituiertes) Benzylamino, C₁₋₄-Alkyl oder C₁₋₄-Alkylsulfid ist; beispielsweise Phenylamino oder 4-Fluorphenylamino;
(iii) R₁₀ C₁₋₄-Alkyl, Methylcarbonyl, Hydroxyethyl, Carbonsäure, Sulfonamid, (optional halogen- oder hydroxy-substituiertes) Phenyl (optional halogen- oder hydroxy-substituiertes) Pyridyl (beispielsweise 6-Fluorpyrid-2-yl) oder Thiadiazolyl (z. B. 1,2,3-Thiadiazol-4-yl) ist; und
(iv) X und Y unabhängig voneinander C oder N sind,
in freier oder pharmazeutisch verträglicher Salzform, einschließlich seiner Enantiomere, Diastereomere und Racemate;
**(C) Formel II:** 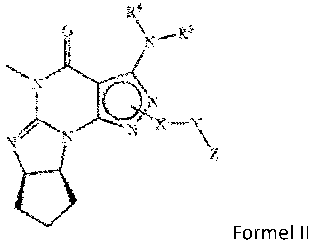
(i) X ist C₁₋₆-Alkylen (z. B. Methylen, Ethylen oder Prop-2-yn-1-ylen);
(ii) Y ist eine Einfachbindung, Alkinylen (z. B. -C≡C-), Arylen (z. B. Phenylen) oder Heteroarylen (z. B. Pyridylen);
(iii) Z ist H, Aryl (z. B. Phenyl), Heteroaryl (z. B. Pyridyl, z. B. Pyrid-2-yl), Halogen (z. B. F, Br, CI), Halogen-C₁₋₆-Alkyl (z. B. Trifluormethyl), -C(O)-R¹, -N(R²)(R³), oder C₃₋₇₋-Cycloalkyl, optional mit mindestens einem Atom aus einer Gruppe bestehend aus N oder O (z. B. Cyclopentyl, Cyclohexyl, Tetrahydro-2H-pyran-4-yl oder Morpholinyl);
(iv) R¹ ist C₁₋₆-Alkyl, Halogen-C₁₋₆-Alkyl, -OH oder -OC₁₋₆-Alkyl (z. B. -OCH₃);
(v) R² und R³ sind unabhängig voneinander H- oder C₁₋₆-Alkyl;
(vi) R⁴ und R³ sind unabhängig voneinander H, C₁₋₆-Alkyl oder Aryl (z. B. Phenyl), optional mit einem oder mehreren Halogenen (z. B. Fluorphenyl, z. B. 4-Fluorphenyl), Hydroxy (z. B. Hydroxyphenyl, z. B. 4-Hydroxyphenyl oder 2-Hydroxyphenyl) oder C₁₋₆-Alkoxy substituiert;
(vii) wobei X, Y und Z unabhängig und optional mit einem oder mehreren Halogenen (z. B. F, Cl oder Br), C₁₋₆-Alkyl (z. B. Methyl), Halogen-C₁₋₆-Alkyl (z. B. Trifluormethyl) substituiert sind, beispielsweise Z Heteroaryl ist, z. B. Pyridyl, substituiert mit einem oder mehreren Halogenen (z. B. 6-Fluorpyrid-2-yl, 5-Fluorpyrid-2-yl, 6-Fluorpyrid-2-yl, 3-Fluorpyrid-2-yl, 4-Fluorpyrid-2-yl, 4,6-Dichlorpyrid-2-yl), Halogen-C₁₋₆-Alkyl (z. B. 5-Trifluormethylpyrid-2-yl) oder C₁₋₆-Alkyl (z. B. 5-Methylpyrid-2-yl), oder Z ist Aryl, z. B. Phenyl, substituiert mit einem oder mehreren Halogenen (z. B. 4-Fluorphenyl).
in freier oder Salzform;
**(D) Formel III:** 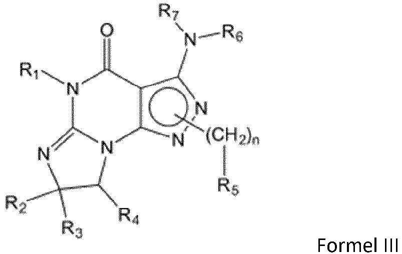 wobei
(i) R1 H oder C₁₋₄-Alkyl (z. B. Methyl oder Ethyl) ist;
(ii) R₂ und R₃ unabhängig voneinander H oder C₁₋₆-Alkyl (z. B. Methyl oder Ethyl) sind;
(iii) R₄ H oder C₁₋₄-Alkyl (z.B. Methyl oder Ethyl) ist;
(iv) R₅ Aryl (z. B. Phenyl) ist, optional mit einer oder mehreren Gruppen substituiert, unabhängig voneinander ausgewählt aus -C(=O)-C₁₋₆-Alkyl (z.B. -C(=O)-CH₃) und C₁₋₆-Hydroxyalkyl (z. B. 1-Hydroxyethyl);
(v) R₆ und R₇ unabhängig voneinander H oder Aryl (z. B. Phenyl) sind, optional mit einer oder mehreren Gruppen substituiert, unabhängig voneinander ausgewählt aus C₁₋₆-Alkyl (z. B. Methyl oder Ethyl) und Halogen (z. B. F oder CI), beispielsweise unsubstituiertes Phenyl oder Phenyl substituiert mit einem oder mehreren Halogenen (z. B. F) oder Phenyl substituiert mit einem oder mehreren C₁₋₆-Alkyl und einem oder mehreren Halogenen oder Phenyl substituiert mit einem C₁₋₆-Alkyl und einem Halogen, beispielsweise 4-Fluorphenyl oder 3,4-Difluorphenyl oder 4-Fluor-3-methylphenyl; und
(vi) n 1, 2, 3 oder 4 ist,
in freier oder Salzform; und
**(E) Formel IV** 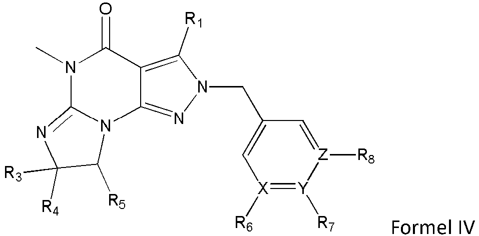 wobei
(i) R₁ C₁₋₄-Alkyl (z. B. Methyl oder Ethyl) oder -NH(R₂) ist, wobei R₂ Phenyl ist, optional mit einem Halogen (z. B. Fluor) substituiert, beispielsweise 4-Fluorphenyl;
(ii) X, Y und Z unabhängig voneinander N oder C sind;
(iii) R₃, R₄ und R₅ unabhängig voneinander H oder C₁₋₄-Alkyl (z. B. Methyl) sind; oder R₃ H ist und R₄ und R₅ zusammen eine Trimethylenbrücke bilden (wobei vorzugsweise R₄ und R₅ zusammen die cis-Konfiguration aufweisen, z. B. wobei die kohlenstoffhaltigen R₄ und R₅ die R- bzw. S-Konfiguration aufweisen),
(iv) R₆, R₇ und R₈ unabhängig voneinander sind:
H,
C₁₋₄-Alkyl (z. B. Methyl),
Pyrid-2-yl substituiert mit Hydroxy, oder
-S(O)₂-NH₂;
(iv) vorausgesetzt, dass wenn X, Y und/oder Z N sind, dann R₆, R₇ und/oder R₈ jeweils nicht vorhanden sind; und wenn X, Y und Z alle C sind, dann mindestens einer von R₆, R₇ oder R₈ -S(O)₂-NH₂ oder Pyrid-2-yl, substituiert mit Hydroxy ist,
in freier oder Salzform.

2. Inhibitor oder Zusammensetzung zur Verwendung nach Anspruch 1, wobei der Patient aufweist
a. erhöhte Konzentrationen eines oder mehrerer proinflammatorischer Zytokine (z. B. ausgewählt aus IL1β, TNFα, Ccl2, IL-6 und Kombinationen davon);
b. reduzierte Konzentrationen eines oder mehrerer entzündungshemmender Zytokine (z. B. IL-10);
c. erhöhte Konzentrationen von Makrophagen des M1-Phänotyps im Vergleich zu Makrophagen des M2-Phänotyps.

3. Inhibitor oder Zusammensetzung zur Verwendung nach Anspruch 1 oder Anspruch 2, wobei der PDE1-Inhibitor folgendermaßen ist: 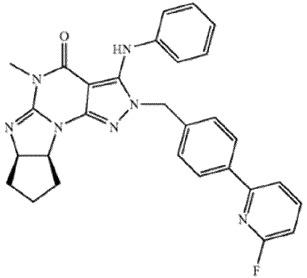 in freier oder pharmazeutisch verträglicher Salzform.

4. Inhibitor oder Zusammensetzung zur Verwendung nach einem vorstehenden Anspruch, wobei der PDE1-Inhibitor folgendermaßen ist: 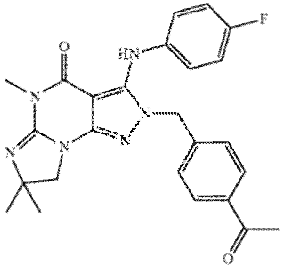 in freier oder pharmazeutisch verträglicher Salzform.

5. Inhibitor oder Zusammensetzung zur Verwendung nach einem vorstehenden Anspruch, wobei der PDE1-Inhibitor folgendermaßen ist: 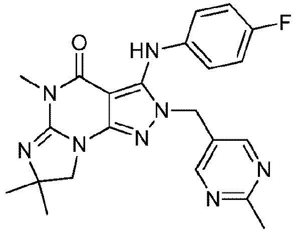 in freier oder pharmazeutisch verträglicher Salzform.

6. Inhibitor oder Zusammensetzung zur Verwendung nach einem vorstehenden Anspruch, wobei der PDE1-Inhibitor folgendermaßen ist: 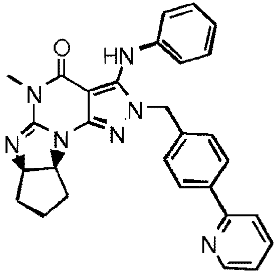 in freier oder pharmazeutisch verträglicher Salzform.

7. Inhibitor oder Zusammensetzung zur Verwendung nach einem vorstehenden Anspruch, wobei der PDE1-Inhibitor in Kombination mit einem PDE4-Inhibitor (z. B. Rolipram) verabreicht wird.

## Revendications

1. Inhibiteur de la PDE1 ou composition pharmaceutique comprenant un inhibiteur de la PDE1 destiné à être utilisé dans un procédé visant à favoriser la résolution de l'inflammation pour le traitement ou la prophylaxie de l'inflammation ou d'une maladie associée à l'inflammation, le procédé comprenant l'administration de l'inhibiteur de la PDE1 ou de la composition pharmaceutique à un patient qui en a besoin, dans lequel le patient souffre d'une maladie ou d'un trouble médié par les macrophages, sélectionné parmi les infections bactériennes à *Salmonella typhi, Salmonella typhimurium, Listeria monocytogenes, Mycobacterium tuberculosis, Mycobacterium ulceranset Mycobacterium avium ;* des infections virales sélectionnées parmi le virus de la peste porcine africaine, le virus de la peste porcine classique, le virus de la dengue, le virus de la fièvre aphteuse, le virus de la grippe A, le circovirus porcin de type 2, le virus du syndrome reproducteur et respiratoire porcin, le virus de la pseudorage porcine, le virus respiratoire syncytial, le coronavirus du syndrome respiratoire aigu sévère, le virus du Nil occidental, les hépatites virales (par exemple, hépatite A, hépatite B, hépatite C) ; les infestations parasitaires (par exemple, *Taenia crassiceps, Toxoplasma gondii, Leishmania infantum, Schistosoma mansoni ) ;* la dermatite atopique ; la pneumonie ; l'obésité et la résistance à l'insuline ; l'asthme ; la fibrose pulmonaire ; la bronchopneumopathie chronique obstructive (BPCO) ; le diabète ; la septicémie ; la stéatoheptatite non alcoolique (NASH) ; l'hépatite auto-immune ; le lupus érythémateux systémique (LES) ; la cicatrisation des plaies ; la pleurésie ; la péritonite ; et la fibrose kystique, et
dans lequel l'inhibiteur de la PDE1 est un composé sélectionné parmi
**(A) Formule I** : 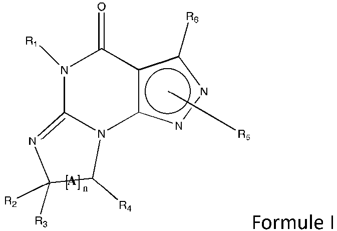 dans laquelle
(i) R₁ est un H ou un alkyle en C₁₋₄ (par exemple, un méthyle) ;
(ii) R₄ est un H ou un alkyle en C₁₋₄ et R₂ et R₃ sont, indépendamment, un H ou un alkyle en C₁₋₄ (par exemple, R₂ et R₃ sont tous deux des groupes méthyle, ou R₂ est un H et R₃ est un isopropyle), un aryle, hétéroaryle, (éventuellement hétéro)arylalcoxy, ou (éventuellement hétéro)arylalkyle ; ou
R₂ est un H et R₃ et R₄ forment ensemble un pont di-, tri- ou tétraméthylène (de préférence dans lequel R₃ et R₄ présentent ensemble la configuration *cis,* par exemple, dans lequel les carbones portant R₃ et R₄ présentent respectivement les configurations R et S) ;
(iii) R₅ est un hétéroarylalkyle substitué, par exemple, substitué par un haloalkyle ;
ou R₅ est lié à l'un des atomes d'azote de la partie pyrazole de la formule I et constitue un fragment de la formule A 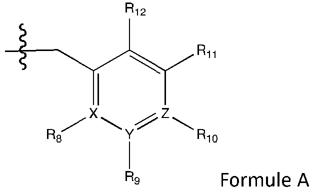
dans lequel X, Y et Z sont, indépendamment, N ou C, et R₈, R₉, R₁₁ et R₁₂ sont indépendamment un H ou halogène (par exemple, Cl ou F), et R₁₀ est un halogène, un groupe alkyle, cycloalkyle, haloalkyle (par exemple, trifluorométhyle), aryle (par exemple, phényle), hétéroaryle (par exemple, pyridyle (par exemple, pyrid-2-yle) éventuellement substitué par un halogène, ou thiadiazolyle (par exemple, 1,2,3-thiadiazol-4-yle)), diazolyle, triazolyle, tétrazolyle, arylcarbonyle (par exemple, benzoyle), alkylsulfonyle (par exemple, méthylsulfonyle), hétéroarylcarbonyle ou alcoxycarbonyle ; étant entendu que lorsque X, Y ou Z est de l'azote, R₈, R₉ou R₁₀, respectivement, n'est pas présent ; et
(iv) R₆ est un H, alkyle, aryle, hétéroaryle, arylalkyle (par exemple, benzyle), arylamino (par exemple, phénylamino), hétéroarylamino, N,N-dialkylamino, N,N-diarylamino ou N-aryl-N-(arylalkyl)amino (par exemple, N-phényl-N-(1,1'-biphén-4-ylméthyl)amino) ; et
(v) n=0 ou 1 ;
(vi) quand n=1, A est un -C(R₁₃R₁₄)-
dans laquelle R₁₃ et R₁₄ sont, indépendamment, un H ou un alkyle en C₁₋₄, un aryle, hétéroaryle, (éventuellement hétéro)arylalcoxy ou (éventuellement hétéro)arylalkyle ;
sous forme libre ou de sel, y compris ses énantiomères, diastéréoisomères et racémates ;
**(B) Formule la** : 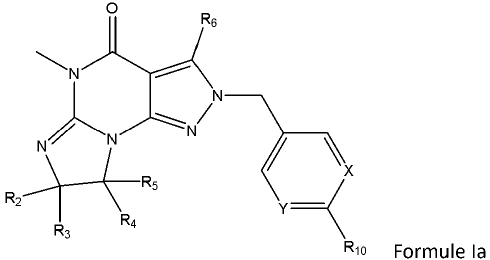 dans laquelle
(i) R₂ et R₅ sont indépendamment un H ou un hydroxy, et R₃ et R₄ forment ensemble un pont tri- ou tétra-méthylène. [pref. les carbones portant R₃ et R₄ présentant la configuration R et S respectivement] ; ou R₂ et R₃ sont chacun un groupe méthyle et R₄ et R₅ sont chacun un H ; ou R₂, R₄ et R₅ sont un H et R₃ est un isopropyle [pref. le carbone portant le R₃ présentant la configuration R] ;
(ii) R₆ est un phénylamino (éventuellement halo- ou hydroxy-substitué), un benzylamino (éventuellement halo- ou hydroxy-substitué), un alkyle en C₁₋₄ou un sulfure d'alkyle en C₁₋₄; par exemple, un phénylamino ou un 4-fluorophénylamino ;
(iii) R₁₀ est un groupe alkyle en C₁₋₄, un méthylcarbonyle, hydroxyéthyle, acide carboxylique, sulfonamide, phényle (éventuellement halogéné ou hydroxylé), pyridyle (éventuellement halogéné ou hydroxylé) (par exemple 6-fluoropyrid-2-yle) ou thiadiazolyle (par exemple 1,2,3-thiadiazol-4-yle) ; et
(iv) X et Y sont indépendamment C ou N,
sous forme libre ou sous forme de sel pharmaceutiquement acceptable, y compris ses énantiomères, diastéréoisomères et racémates ;
**(C) Formule Il** : 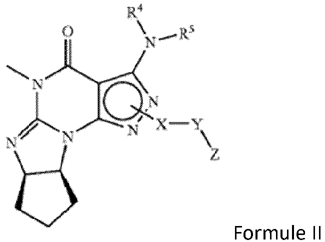
(i) X est un alcylène en C₁₋₆(par exemple, méthylène, éthylène ou prop-2-yn-1-ylène) ;
(ii) Y est une liaison simple, un alcynylène (par exemple, -C=C-), un arylène (par exemple, le phénylène) ou un hétéroarylène (par exemple, le pyridylène) ;
(iii) Z est un H, aryle (par exemple, phényle), hétéroaryle (par exemple, pyridyle, par exemple, pyrid-2-yle), halo (par exemple, F, Br, Cl), haloalkyle en C₁₋₆ (par exemple, trifluorométhyle), -C(O)-R¹, -N(R²)(R³), ou cycloalkyle en C₃₋₇ contenant éventuellement au moins un atome choisi parmi un groupe constitué de N ou O (par exemple, cyclopentyle, cyclohexyle, tétrahydro-2H-pyran-4-yle ou morpholinyle) ;
(iv) R¹ est un groupe alkyle en C₁₋₆, un halo-alkyle en C₁₋₆, un -OH ou -OC₁₋₆ (par exemple, -OCH₃) ;
(v) R² et R³ sont indépendamment un H ou un alkyle en C₁₋₆ ;
(vi) R⁴ et R⁵ sont indépendamment un H, un alkyle en C₁₋₆ ou aryle (par exemple, phényle) éventuellement substitués par un ou plusieurs halo (par exemple, fluorophényle, par exemple, 4-fluorophényle), hydroxy (par exemple, hydroxyphényle, par exemple, 4-hydroxyphényle ou 2-hydroxyphényle) ou alcoxy en C₁₋₆;
(vii) dans laquelle X, Y et Z sont indépendamment et éventuellement substitués par un ou plusieurs halogénures (par exemple, F, CI ou Br), groupes alkyles en C₁₋₆ (par exemple, méthyle), halo-alkyle en C₁₋₆(par exemple, trifluorométhyle), par exemple, Z est un hétéroaryle, par exemple un pyridyle substitué par un ou plusieurs halogénures (par exemple, 6-fluoropyrid-2-yle, 5-fluoropyrid-2-yle, 6-fluoropyrid-2-yle, 3-fluoropyrid-2-yle, 4-fluoropyrid-2-yle, 4,6-dichloropyrid-2-yle), halo-alkyles en C₁₋₆(par exemple, 5-trifluorométhylpyrid-2-yle) ou halo-alkyles en C₁₋₆(par exemple, 5-méthylpyrid-2-yle), ou Z est un aryle, par exemple un phényle, substitué par un ou plusieurs halogénures (par exemple, 4-fluorophényle),
sous forme libre ou de sel ;
**(D) Formule III** : 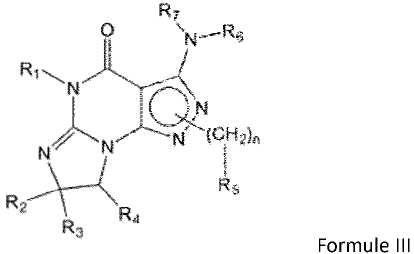 dans laquelle
(i) R1 est un H ou un alkyle en C₁₋₄ (par exemple, méthyle ou éthyle) ;
(ii) R₂ et R₃ sont indépendamment H ou C₁₋₆ alkyl (par exemple, méthyle ou éthyle) ;
(iii) R₄ est un H ou un alkyle en C₁₋₄ (par exemple, méthyle ou éthyle) ;
(iv) R₅ est un groupe aryle (par exemple, phényle) éventuellement substitué par un ou plusieurs groupes choisis indépendamment parmi -C(=O)-alkyle en C₁₋₆ (par exemple, -C(=O)-CH₃) et hydroxyalkyle en C₁₋₆ (par exemple, 1-hydroxyéthyle) ;
(v) R₆ et R₇ sont indépendamment un H ou un aryle (par exemple, phényle) éventuellement substitués par un ou plusieurs groupes indépendamment choisis parmi les groupes alkyle en C₁₋₆ (par exemple, méthyle ou éthyle) et halogène (par exemple, F ou Cl), par exemple phényle non substitué ou phényle substitué par un ou plusieurs halogènes (par exemple, F) ou phényle substitué par un ou plusieurs groupes alkyle en C₁₋₆ et un ou plusieurs halogènes ou phényles substitués par un groupe alkyle en C₁₋₆ et un halogène, par exemple 4-fluorophényle ou 3,4-difluorophényle ou 4-fluoro-3-méthylphényle ; et
(vi) n est égal à 1, 2, 3 ou 4,
sous forme libre ou de sel ; et
**(E) Formule IV** 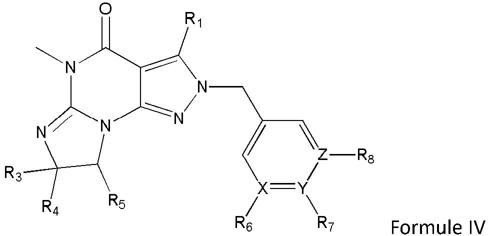 dans laquelle
(i) R₁ est un alkyle en C₁₋₄ (par exemple, méthyle ou éthyle), ou -NH(R₂), dans lequel R₂ est un phényle éventuellement substitué par un halo (par exemple, fluoro), par exemple, 4-fluorophényle ;
(ii) X, Y et Z sont, indépendamment, un N ou C ;
(iii) R₃, R₄ et R₅ sont indépendamment un H ou un alkyle en C₁₋₄ (par exemple, méthyle) ; ou R₃ est un H et R₄ et R₅ forment ensemble un pont triméthylène (de préférence dans laquelle R₄ et R₅ présentent ensemble la configuration cis, par exemple, dans laquelle les carbones portant R₄ et R₅ présentent respectivement les configurations R et S),
(iv) R₆, R₇ et R₅ sont indépendamment :
un H,
un alkyle en C₁₋₄ (par exemple, méthyle),
un pyrid-2-yle substitué par un hydroxy, ou
-S(O)₂-NH₂ ;
(iv) À condition que lorsque X, Y et/ou Z sont un N, alors R₆, R₇ et/ou R₈, respectivement, ne sont pas présents ; et lorsque X, Y et Z sont tous un C, alors au moins un de R₆, R₇ ou R₈ est -S(O)₂-NH₂ ou un pyrid-2-yle substitué par un hydroxy,
sous forme libre ou de sel.

2. Inhibiteur ou composition pour l'utilisation selon la revendication 1, dans lequel le patient présente
a. des niveaux élevés d'une ou plusieurs cytokines pro-inflammatoires (par exemple, sélectionnées parmi IL1β, TNFα, Ccl2, IL-6 et leurs combinaisons) ;
b. des niveaux réduits d'une ou plusieurs cytokines anti-inflammatoires (par exemple, IL-10) ;
c. des niveaux élevés de macrophages de phénotype M1 par rapport aux macrophages de phénotype M2.

3. Inhibiteur ou composition pour l'utilisation selon la revendication 1 ou la revendication 2, dans lequel l'inhibiteur de la PDE1 est le suivant : 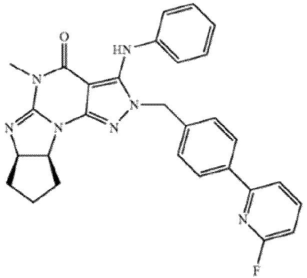 sous forme libre ou sous forme de sel pharmaceutiquement acceptable.

4. Inhibiteur ou composition destinée à être utilisée selon une quelconque revendication précédente, dans lequel l'inhibiteur de la PDE1 est le suivant : 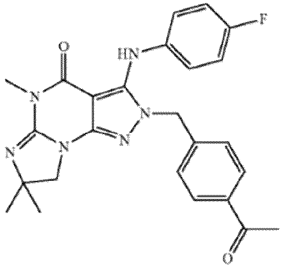 sous forme libre ou sous forme de sel pharmaceutiquement acceptable.

5. Inhibiteur ou composition destinée à être utilisée selon une quelconque revendication précédente, dans lequel l'inhibiteur de la PDE1 est le suivant : 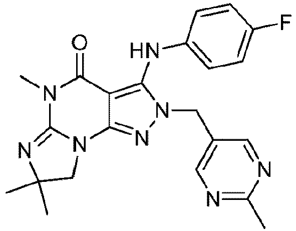 sous forme libre ou sous forme de sel pharmaceutiquement acceptable.

6. Inhibiteur ou composition destinée à être utilisée selon une quelconque revendication précédente, dans lequel l'inhibiteur de la PDE1 est le suivant : 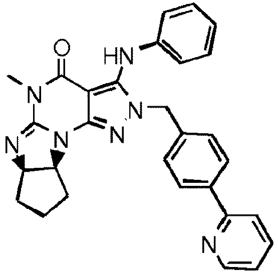 sous forme libre ou sous forme de sel pharmaceutiquement acceptable.

7. Inhibiteur ou composition destinée à être utilisée selon l'une quelconque des revendications précédentes, dans lequel l'inhibiteur de la PDE1 est administré en combinaison avec un inhibiteur de la PDE4 (par exemple, le rolipram).
